# EUROPEAN PATENT APPLICATION

(11) **EP 4 261 657 A1**
(43) Date of publication of application: **18.10.2023**
(21) Application number: 21903215.8
(22) Date of filing: 29.11.2021
(51) Int. Cl.: G06F 3/01

(54) **INFORMATION PROCESSING DEVICE, INFORMATION PROCESSING METHOD, AND COMPUTER-READABLE RECORDING MEDIUM**

(30) Priority: 08.12.2020 JP 2020203354
(71) Applicant: Sony Group Corporation, Tokyo 108-0075 (JP)
(72) Inventor: KASAHARA, Shunichi, Tokyo 108-0075 (JP); TAJIMA, Daisuke, Tokyo 108-0075 (JP)
(74) Representative: MFG Patentanwälte Meyer-Wildhagen Meggle-Freund Gerhard PartG mbB
(86) International application number: PCT/JP2021/043587
(87) International publication number: WO 2022/124111

(57) **Abstract**

An information processing apparatus according to an embodiment of the present technology includes a control unit. The control unit controls an action unit at a third point of time on the basis of a signal indicating a voluntary movement of a user at a first point of time, the action unit being attached to a body part of the user and moving the body part of the user, the third point of time being included in a period from the first point of time to a second point of time, the second point of time being a point of time when a movement of the body part of the user according to the signal indicating the voluntary movement of the user occurs.

## Description

### Technical Field

The present technology relates to an information processing apparatus, an information processing method, and a computer-readable recording medium that can be applied to an apparatus for driving a body to assist a movement.

### Background Art

Conventionally, a method of moving the body of a user to make a body movement has been developed. For example, known methods for making a movement use electrical muscle stimulation (EMS) and a mechanical actuator for driving joints. The use of these methods allows assistance and the like of various movements.

For example, Non-Patent Literature 1 has described a system for accelerating a human reaction using EMS. This system is configured to make a body movement using the EMS in advance during a particular period in which a user receives a visual stimulation and reacts it, for example. Setting an appropriate timing to make the body movement using this EMS allows assistance of the movement of the user with less deterioration of the user's sensation of making the movement himself or herself.

### Citation List

### Patent Literature

Non-Patent Literature 1: Shunichi Kasahara, Jun Nishida, and Pedro Lopes. "Preemptive Action: Accelerating Human Reaction using Electrical Muscle Stimulation Without Compromising Agency." In CHI Conference on Human Factors in Computing Systems Proceedings (CHI 2019), Glasgow, Scotland UK. ACM, New York, NY, USA. May 4-9, 2019. Paper No. 643, 15 pages

### Disclosure of Invention

### Technical Problem

In the human movement assistance as described above, a user's movement timing can be incorrect depending on an assistance timing. Therefore, it is desirable to provide a technology capable of suitably controlling a user's movement timing.

In view of the above-mentioned circumstances, it is an objective of the present technology to provide an information processing apparatus, an information processing method, and a computer-readable recording medium that are capable of suitably controlling a user's movement timing.

### Solution to Problem

In order to accomplish the above-mentioned objective, an information processing apparatus according to an embodiment of the present technology includes a control unit.

The control unit controls an action unit at a third point of time on the basis of a signal indicating a voluntary movement of a user at a first point of time, the action unit being attached to a body part of the user and moving the body part of the user, the third point of time being included in a period from the first point of time to a second point of time, the second point of time being a point of time when a movement of the body part of the user according to the signal indicating the voluntary movement of the user occurs.

In this information processing apparatus, moving the action unit attached to the body part assists the movement of the body part of the user that occurs at the second point of time in accordance with the signal indicating the voluntary movement of the user at the first point of time. The action unit is driven at the third point of time in the period from the first point of time to the second point of time. This enables assistance to start preceding the movement of the body part of the user. It can suitably control the user's movement timing.

The control unit may detect a movement signal for making the voluntary movement of the user from the signal indicating the voluntary movement of the user and set the third point of time to control the action unit, using a detection timing of the movement signal as a basis.

The control unit may control, in a case where the movement signal has been detected, the action unit to accelerate or decelerate the movement of the body part of the user, using as the second point of time as a basis.

The control unit may control the action unit on the basis of a reference signal for the movement of the body part of the user.

The reference signal may be a signal indicating a timing of the movement of the body part of the user. In this case, the control unit may set, on the basis of the timing of the movement of the body part of the user, at least one of the third point of time to control the action unit or a fourth point of time to terminate control on the action unit.

The control unit may set at least one of the third point of time or the fourth point of time in accordance with a driving force to drive the action unit.

The action unit may be capable of driving to promote or suppress the movement of the body part of the user. In this case, the control unit may set the third point of time to be later as the driving force of the action unit increases in a case of promoting the movement of the body part of the user and set the fourth point of time to be earlier as the driving force of the action unit increases in a case of suppressing the movement of the body part of the user.

The reference signal may be a signal indicating necessity or unnecessity of a predetermined movement that the user performs by moving the body part of the user. In this case, the control unit may compare necessity or unnecessity of the predetermined movement indicated by the reference signal with presence or absence of the movement signal for making the predetermined movement and control the action unit on the basis of the comparison result.

The control unit may control the action unit to cause the body part of the user to perform the predetermined movement in a case where the movement signal for making the predetermined movement has not been detected in a situation where the predetermined movement is necessary.

The control unit may control the action unit to prevent the body part of the user from performing the predetermined movement in a case where the movement signal for making the predetermined movement has been detected in a situation where the predetermined movement is unnecessary.

The reference signal may be a signal indicating a result of sensing information for the user to determine whether or not to move the body part of the user, the sensing being performed through a predetermined sensor.

The movement of the body part of the user may be a movement for performing an input operation for a predetermined application. In this case, the reference signal may be a signal indicating a timing of the input operation.

The reference signal may be a movement signal detected from a signal indicating a voluntary movement of another user different from the user.

The signal indicating the voluntary movement of the user may be at least one of an electromyography signal of the user, an electroencephalography signal of the user, or a spinal cord signal of the user.

The action unit may be a first magnetic field action unit that receives a force depending on a magnetic field. In this case, the control unit may control the first magnetic field action unit by electrically changing a magnetic field between the first magnetic field action unit and a second magnetic field action unit provided separate from the first magnetic field action unit.

At least one of the first magnetic field action unit or the second magnetic field action unit may include an electromagnet. In this case, the control unit may control the electromagnet to generate a magnetic force between the first magnetic field action unit and the second magnetic field action unit, the magnetic force being attraction or repulsion.

The movement of the body part of the user may be a contact movement in which the user touches a movement target with the body part of the user. In this case, the second magnetic field action unit may be provided in the movement target.

The body part of the user may be a finger of the user. In this case, the contact movement may be a contact operation with the finger of the user. Moreover, the movement target may be an input apparatus that receives the contact operation.

An information processing method according to an embodiment of the present technology includes
by a computer system,
controlling an action unit at a third point of time on the basis of a signal indicating a voluntary movement of a user at a first point of time, the action unit being attached to a body part of the user and moving the body part of the user, the third point of time being included in a period from the first point of time to a second point of time, the second point of time being a point of time when a movement of the body part of the user according to the signal indicating the voluntary movement of the user occurs.

A computer-readable recording medium according to an embodiment of the present technology records a program that causes a computer system to execute the following step.

A step of controlling an action unit at a third point of time on the basis of a signal indicating a voluntary movement of a user at a first point of time, the action unit being attached to a body part of the user and moving the body part of the user, the third point of time being included in a period from the first point of time to a second point of time, the second point of time being a point of time when a movement of the body part of the user according to the signal indicating the voluntary movement of the user occurs.

### Brief Description of Drawings

[Fig. 1] A schematic diagram showing a configuration example of a body driving system according to a first embodiment of the present technology.
[Fig. 2] A block diagram showing functional configuration examples of the body driving system shown in Fig. 1.
[Fig. 3] A graph showing an example of an electromyography signal.
[Fig. 4] A schematic diagram showing basic movement assistance of a body driving unit.
[Fig. 5] A schematic diagram for describing a basic operation of the body driving system.
[Fig. 6] A schematic diagram for describing a start timing of movement assistance.
[Fig. 7] A block diagram showing a flow of movement assistance with no reference signal.
[Fig. 8] A flowchart showing an example of the reference signal with no movement assistance.
[Fig. 9] A schematic diagram showing an example of movement assistance to accelerate or decelerate a movement of a finger.
[Fig. 10] A block diagram showing a flow of movement assistance with a reference signal.
[Fig. 11] A flowchart showing an example of movement assistance with the reference signal.
[Fig. 12] A schematic diagram showing an example of movement assistance to control a timing of a contact operation.
[Fig. 13] A graph showing control contents of the movement assistance shown in Fig. 12.
[Fig. 14] A schematic diagram showing an example of movement assistance in conjunction with another user.
[Fig. 15] A block diagram showing a flow of the movement assistance shown in Fig. 14.
[Fig. 16] A schematic diagram for describing control contents of the movement assistance shown in Fig. 14.
[Fig. 17] A schematic diagram showing an example of movement assistance for a movement involving a cognitive judgement.
[Fig. 18] A schematic diagram showing a configuration example of a body driving system according to a second embodiment.
[Fig. 19] A schematic diagram showing a configuration example of a body driving system according to a third embodiment.

### Mode(s) for Carrying Out the Invention

Hereinafter, embodiments according to the present technology will be described with reference to the drawings.

### <First Embodiment>

### [Configuration of Body Driving System]

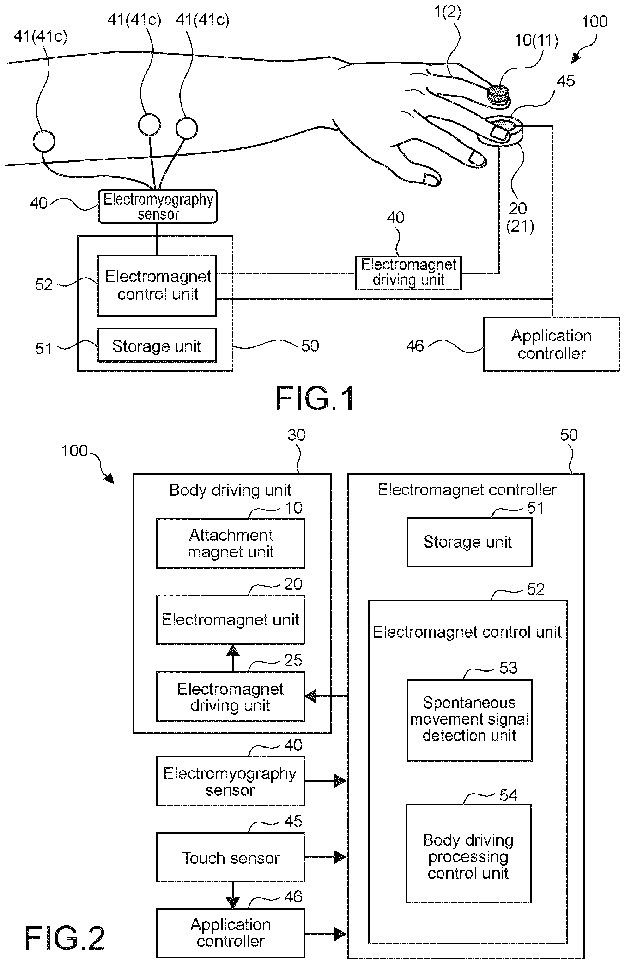

Fig. 1 is a schematic diagram showing a configuration example of a body driving system according to a first embodiment of the present technology.

A body driving system 100 is a system that achieves movement assistance for assisting a movement of a body part that is a body part of a user 1. The body driving system 100 performs, for example, movement assistance to promote the movement of the body part or movement assistance to suppress the movement of the body part, for example, so as to appropriately perform the movement of the user 1.

The body driving system 100 achieves the movement assistance by moving an action unit attached to the body part. Configuring the action unit to be capable of moving a body part, e.g., a finger 2 of a hand, a palm, an arm, a leg, a body, or a head of the user 1 can assist a movement of each body part.

In the present embodiment, the body driving system 100 performs movement assistance for assisting a movement in which the user 1 touches a movement target with the body part. That is, the movement of the user 1 of the body part as a movement assistance target is a contact movement in which the user 1 touches the movement target with the body part of the user 1. Here, the contact movement is a series of movements involving contact between the user 1 and the target, for example. The contact movement includes a movement, e.g., pressing a button, touching a screen, or grasping an object.

It should be noted that the body driving system 100 has movement assistance targets other than the contact movement. For example, the body driving system 100 may assist a movement that the user 1 performs with a tool (e.g., a movement of the back of the hand (wrist) of the user 1 when using a pen) or a movement that needs force control (e.g., a movement of moving fingers 2 slightly upward when making pottery). Thus, the body driving system 100 can also assist a movement that causes no contact with a target.

The body driving system 100 shown in Fig. 1 is configured to assist a contact operation in which the user 1 touches a movement target (a touch sensor 45, which will be described later) with his or her finger 2. The contact operation is, for example, a movement that the user 1 performs by touching the movement target with the finger 2.

In the present embodiment, the finger 2 of the user 1 is an example of the body part of the user 1. Moreover, the contact operation with the finger of the user 1 is an example of the above-mentioned contact operation.

The body driving system 100 moves the finger 2 of the user 1 through an attachment magnet unit 10 and an electromagnet unit 20.

The attachment magnet unit 10 is attached to the user 1 for use. The attachment magnet unit 10 includes an attachment magnet 11 attached to the finger 2 of the user 1. The electromagnet unit 20 includes an electromagnet 21. The electromagnet 21 generates a magnetic field that acts on the attachment magnet 11.

In Fig. 1, the attachment magnet 11 is attached to an index finger of the right hand of the user 1. In this case, movement assistance for assisting a contact operation by the index finger of the right hand is performed. As a matter of course, the attachment magnet 11 may be attached to another finger 2 of the user 1. The attachment magnet unit 10 is an example of the above-mentioned action unit.

For example, controlling a current input to the electromagnet 21 generates a magnetic force (attraction) to attract the attachment magnet 11 or a magnetic force (repulsion) to repel the attachment magnet 11. Such a magnetic force (attraction or repulsion) that acts on the attachment magnet 11 moves the finger 2 of the user 1 to which the attachment magnet 11 has been attached.

Moreover, the body driving system 100 monitors a signal indicating a voluntary movement to move the finger 2 as a movement assistance target.

The voluntary movement of the user 1 is, for example, a movement that the user 1 voluntarily performs. For example, an action achieved by a voluntary movement to move each body part is the movement of the user 1. A voluntary movement to bend each joint of the finger 2 achieves a movement intended by the user 1, for example, in a case of performing a movement in which the user 1 touches the target with the finger 2.

The signal indicating the voluntary movement is a biological signal that varies depending on the voluntary movement, for example. The signal indicating the voluntary movement is a signal indicative of the occurrence of the voluntary movement. For example, in a case where the user 1 performs a voluntary movement, the brain of the user 1 generates a signal for transmitting a command to move a muscle. The signal is transmitted to a target muscle via a neural system such as a spinal cord, such that the muscle contracts. The voluntary movement thus occurs. The signal transmitted in this process is the signal indicating the voluntary movement.

Thus, monitoring the signal indicating the voluntary movement can detect the occurrence of the movement before each body part moves.

In the present embodiment, an electromyography (EMG) signal is used as the signal indicating the voluntary movement. In Fig. 1, an EMG sensor 40 attached to an arm of the user 1 detects an EMG signal for moving a muscle that moves the finger 2 as a movement assistance target. The electromagnet 21 is controlled using the EMG signal detected by the EMG sensor 40.

This enables the electromagnet 21 to be driven to start the movement assistance, for example, before the finger 2 of the user 1 actually moves.

Hereinafter, a specific configuration of the body driving system 100 will be described.

Fig. 2 is a block diagram showing functional configuration examples of the body driving system 100 shown in Fig. 1. The body driving system 100 includes a body driving unit 30, the EMG sensor 40, the touch sensor 45, an application controller 46, and an electromagnet controller 50.

The body driving unit 30 is a mechanism for driving the body of the user 1. The body driving unit 30 includes the attachment magnet unit 10, the electromagnet unit 20, and an electromagnet driving unit 25.

The attachment magnet unit 10 is attached to the finger 2 of the user 1 and moves the finger 3 of the user 1. The attachment magnet unit 10 includes the attachment magnet 11. The attachment magnet 11 is a permanent magnet attached to the finger 2 of the user 1. A typically compact neodymium magnet is used as the attachment magnet 11. It can easily generate relatively strong attraction or repulsion in the finger 2 of the user 1.

Thus, the attachment magnet unit 10 can be considered as an element that receives a force depending on a magnetic field and moves the finger 2 of the user 1. In the present embodiment, the attachment magnet unit 10 corresponds to an action unit. Moreover, the attachment magnet unit 10 functions as a first magnetic field action unit that receives a force depending on a magnetic field.

The attachment magnet 11 is attached to the finger 2 of the user 1 via an attachment tool (not shown), for example. For example, the attachment magnet 11 is fixed to a ring-type attachment tool, a band-type attachment tool, or a bag-type attachment tool. The attachment magnet 11 is disposed on a nail side near the tip of the finger 2 as shown in Fig. 1, for example. This enables the finger 2 to have a sense of touch on a pulp side. Moreover, the attachment magnet 11 is disposed with one magnetic pole (S-pole or N-pole) facing the nail side.

Specific configurations such as type and arrangement of the attachment magnet 11 are not limited. For example, a plurality of attachment magnets 11 may be disposed in a single finger 2. Alternatively, for example, the attachment magnet 11 may be disposed in the side surface or pulp side of the finger 2. Alternatively, the attachment magnet 11 may be directly fixed to each finger 2 of the user 1.

The electromagnet unit 20 includes the electromagnet 21 that generates a magnetic field that acts on the attachment magnet unit 10 (attachment magnet 11). The electromagnet 21 includes, for example, a coil and an iron core wound with the coil. The electromagnet 21 allows a current to flow through the coil to magnetize the iron core and generate a magnetic field. Changing value and orientation of a current flowing through the coil changes the magnetic field. This enables control on orientation and strength of a force that acts on the attachment magnet unit 10.

Thus, the electromagnet unit 20 can be considered as an element that is provided separate from the attachment magnet unit 10 and generates a magnetic field to move the attachment magnet unit 10.

In the present embodiment, the electromagnet unit 20 functions as a second magnetic field action unit.

As shown in Fig. 1, the electromagnet unit 20 is provided in the touch sensor 45 that is a movement target for the finger 2 of the user 1. Specifically, the electromagnet unit 20 is disposed on a side (e.g., a lower side of the contact surface) opposite to a contact surface of the touch sensor 45 that the user 1 touches with the finger 2.

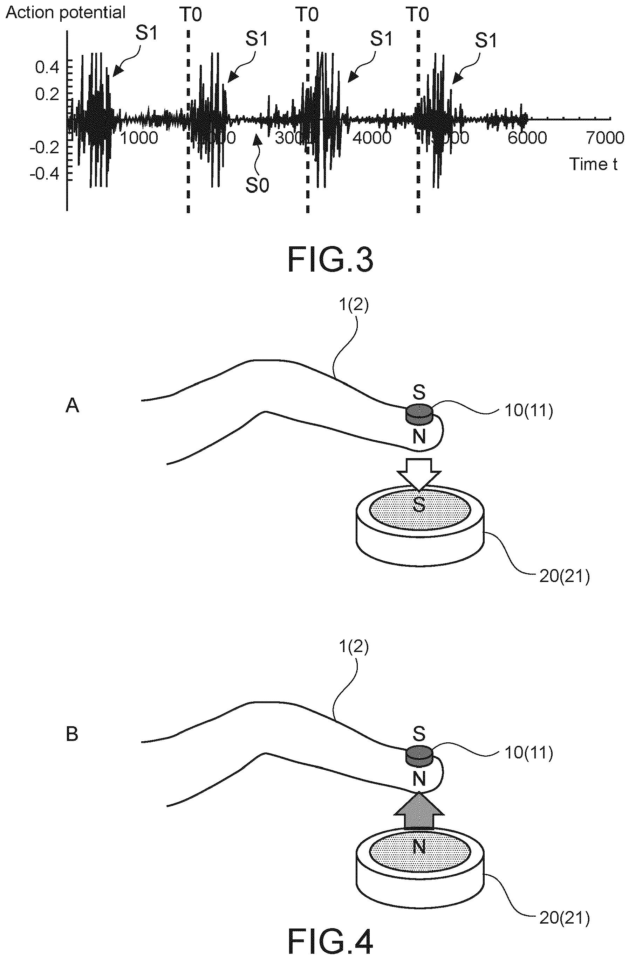

This enables movement assistance, e.g., moving the finger 2 closer to the touch sensor 45 or moving the finger 2 away from the touch sensor 45 (see Fig. 3).

As described above, in the present embodiment, the permanent magnet (attachment magnet 11) is used in the first magnetic field action unit (attachment magnet unit 10) provided on the body side of the user 1 and the electromagnet 21 is provided in the second magnetic field action unit (electromagnet unit 20) provided on the movement target side.

Not limited thereto, for example, the electromagnet may be provided in the first magnetic field action unit on the body side and the permanent magnet may be provided in the second magnetic field action unit on the movement target side. Moreover, for example, electromagnets may be provided both in the first magnetic field action unit on the body side and the second magnetic field action unit on the movement target side.

Thus, at least one of the first magnetic field action unit and the second magnetic field action unit includes the electromagnet. The use of the electromagnet allows easy control on the movement of the body part (finger 2) of the user 1.

The electromagnet driving unit 25 drives the electromagnet 21 provided in the electromagnet unit 20. The electromagnet driving unit 25 generates a control signal according to a driving signal generated by an electromagnet control unit 52 (body driving processing control unit 54), which will be described later, and supplies it to each electromagnet 21. The driving signal may be a digital signal such as a pulse width modulation (PWM) signal or may be an analog current signal.

Specific configurations of the electromagnet driving unit 25 are not limited. For example, the electromagnet driving unit 25 may be configured as appropriate using a current-source capable of driving the electromagnet 21. It should be noted that the electromagnet driving unit 25 may be configured as a part of the electromagnet controller 50.

The EMG sensor 40 is a sensor for detecting an EMG signal for moving a muscle of the user 1. The EMG signal is a signal indicating a change over time of an action potential for moving the muscle. The signal is used as a signal indicating the voluntary movement of the user 1.

In the present embodiment, the EMG sensor 40 that detects a surface EMG is used with a surface electrode 41 attached to a skin surface of the user 1. The use of the surface EMG allows a reduction of the burden on the user 1.

The surface electrode 41 includes a pair of measurement electrodes 41a and 41b and a reference electrode 41c.

The measurement electrodes 41a and 41b are attached to positions where they can detect EMG signals of a muscle for moving a body part (here, the index finger 2 of the right hand) as a movement assistance target. A change in potential between the measurement electrodes 41a and 41b is detected as the EMG signal. The reference electrode 41c is attached to a position apart from the measurement electrodes 41a and 41b. Referring to the potential of the reference electrode 41c can accurately detect a target EMG signal. It should be noted that a configuration excluding the reference electrode 41c may be used.

Fig. 3 is a graph showing an example of the EMG signal. Fig. 3 shows an EMG graph plotting EMG signals (action potentials) along a time axis. The vertical axis of the graph indicates an action potential measured between the measurement electrodes 41a and 41b and the horizontal axis indicates a time.

For example, when the user 1 performs a voluntary movement to move a body part, the action potential changes greatly (positive/negative amplitude increases). Fig. 3 has four waveforms (envelope) that changes in action potential greatly. These waveforms transmit a signal for moving the muscle actually.

Hereinafter, a signal for making the voluntary movement of the user 1, i.e., the signal for moving the muscle actually will be referred to as a spontaneous movement signal S1. The spontaneous movement signal S1 can be considered as a biological signal (living body voluntary movement signal) indicating its voluntary movement.

For example, in the EMG, the spontaneous movement signal S1 (waveform in which the action potential changes greatly) is generated when performing the voluntary movement. Moreover, the EMG signal other than the spontaneous movement signal S1 will be referred to as a steady-state signal S0. In the present embodiment, the spontaneous movement signal S1 corresponds to a movement signal.

It should be noted that as described above, muscle force generation in which the muscle actually contracts occurs after a predetermined time elapses after the action potential (spontaneous movement signal S1) changes. Moreover, a time for muscle contraction (delay time of muscle force generation) after the action potential changes differs depending on kinds of body part and target muscle.

Recording such a delay time in advance allows estimation in advance of a timing when the body part actually moves and the like.

Moreover, the EMG sensor 40 detects an action potential at a predetermined sensing rate (e.g., 1 kHz). The sensing rate is set as appropriate to provide a shorter interval than the delay time of muscle force generation, for example.

Referring back to Fig. 2, the touch sensor 45 is a sensor for detecting contact of the finger 2 of the user 1 with the contact surface. For example, a capacitive contact detection sensor, a pressure sensor, or a mechanical button is used as the touch sensor 45.

The detection result of the touch sensor 45 is output to the application controller 46 or the electromagnet controller 50, which will be described later, as a user operation input associated with the contact operation. Thus, the touch sensor 45 functions as an input apparatus that receives the contact operation by the finger 2 of the user 1.

The application controller 46 is an arithmetic apparatus for operating an application using the touch sensor 45. The application controller 46 is a computer such as a PC and a game device.

Moreover, the application controller 46 is connected to a display apparatus such as a display (not shown) and displays an operation screen or the like of an application (e.g., a rhythm game) using the touch sensor 45.

The application controller 46 executes, for example, various types of processing such as processing of generating an operation screen of an application, processing of changing the operation screen in accordance with the progress of the application, and processing of reflecting an input or the like of a contact operation made by the user 1 on an application operation.

The electromagnet controller 50 is a control unit that controls the operation of the electromagnet 21 of the electromagnet unit 20. The electromagnet controller 50 includes a storage unit 51 and the electromagnet control unit 52.

The storage unit 51 is a nonvolatile storage device. For example, a recording medium using a solid-state element such as a solid state drive (SSD) or a magnetic recording medium such as a hard disk drive (HDD) is used as the storage unit 51. In addition, the type and the like of the recording medium used as the storage unit 51 are not limited. For example, any recording medium for recording non-transitory data may be used as the storage unit 51.

The storage unit 51 stores a control program according to the present embodiment. The control program is a program for controlling the entire operation of the electromagnet controller 50, for example. In addition, information stored in the storage unit 51 is not limited.

In the present embodiment, the storage unit 51 corresponds to a computer-readable recording medium recording a program. Moreover, the control program corresponds to a program recorded on a recording medium.

The electromagnet control unit 52 controls the operation of the electromagnet controller 50. The electromagnet control unit 52 has, for example, hardware configurations necessary for a computer, such as CPU and memories (RAM, ROM). Various types of processing are executed by the CPU loading the control program stored in the storage unit 51 to the RAM and executing it. The electromagnet control unit 52 corresponds to an information processing apparatus according to the present embodiment.

For example, a programmable logic device (PLD) such as a field programmable gate array (FPGA) or another device such as an application specific integrated circuit (ASIC) may be used as the electromagnet control unit 52. Moreover, for example, a processor such as a graphics processing unit (GPU) may be used as the electromagnet control unit 52. Moreover, the electromagnet control unit 52 may be configured using, for example, hardware that configures the above-mentioned application controller 46.

In the present embodiment, a spontaneous movement signal detection unit 53 and a body driving processing control unit 54 are achieved as functional blocks by the CPU of the electromagnet control unit 52 executing the program (control program) according to the present embodiment. Then, these functional blocks execute an information processing method according to the present embodiment. It should be noted that dedicated hardware such as an integrated circuit (IC) may be used as appropriate in order to achieve the respective functional blocks.

The spontaneous movement signal detection unit 53 acquires a signal indicating the voluntary movement of the user 1. Specifically, the EMG sensor 40 sequentially reads EMG signals detected at the predetermined sensing rate.

Moreover, the spontaneous movement signal detection unit 53 detects a spontaneous movement signal S1 from the EMG signal. Specifically, the spontaneous movement signal detection unit 53 detects a point of time when the spontaneous movement signal S1 is generated from a value (action potential) of the EMG signal.

The spontaneous movement signal detection unit 53 determines that the spontaneous movement signal S1 has been generated in a case where the action potential indicated by the EMG signal exceeds a predetermined threshold. Then, the point of time when the spontaneous movement signal S1 has been generated is output as a detection timing T0 of the spontaneous movement signal S1.

Fig. 3 shows the detection timing T0 of the spontaneous movement signal S1 detected from the EMG signal as the dotted line. The detection timing T0 corresponds to a point of time when the waveform of the spontaneous movement signal S1 starts.

The threshold for determining the action potential is set in accordance with, for example, the level or the like of the steady-state signal S0 so as to allow appropriate determination as to the spontaneous movement signal S1, for example.

This enables detection of the signal (spontaneous movement signal S1) for executing a movement before the actual movement occurs. It is because a muscle action potential for the corresponding muscle to generate a muscle force is output before driving the human body.

As described above, the spontaneous movement signal detection unit 53 detects the spontaneous movement signal S1 generated before bending the muscle through the EMG sensor 40.

The body driving processing control unit 54 controls movement assistance (body driving processing) performed by the body driving unit 30.

As described above, the body driving unit 30 drives the attachment magnet 11 with a magnetic field that acts on the attachment magnet 11, i.e., a magnetic field generated by the electromagnet 21. Thus, the body driving processing control unit 54 controls the attachment magnet unit 10 by electrically changing the magnetic field between the attachment magnet unit 10 (attachment magnet 11) and the electromagnet unit 20 (electromagnet 21).

Specifically, the body driving processing control unit 54 generates a control signal for controlling the electromagnet 21. The body driving processing control unit 54 sets, for example, control parameters such as a timing to control the attachment magnet 11, a direction, a period, and a strength. The body driving processing control unit 54 generates a control signal for controlling the electromagnet 21 on the basis of the control parameters. The body driving processing control unit 54 outputs the generated control signal to the electromagnet driving unit 25.

In this manner, the body driving processing control unit 54 controls the magnetic field generated by the electromagnet unit 20 (electromagnet 20), thereby controlling the attachment magnet unit 10 (attachment magnet 11) that is the action unit that moves the body part of the user 1. In the present disclosure, controlling the action unit is controlling the operation of the action unit. Here, the body driving processing control unit 54 controls the timing to control the attachment magnet 11, the direction, the period, the strength, and the like. It should be noted that the timing to control the attachment magnet 11 is a timing to start to control the attachment magnet 11 and a timing to drive the attachment magnet 11.

Fig. 4 is a schematic diagram showing basic movement assistance by the body driving unit 30. Here, the N-pole of the attachment magnet 11 faces the electromagnet 21.

In Fig. 4A, a control signal for causing a current to flow to the electromagnet 21 is generated so that the side of the touch sensor 45 of the electromagnet 21 is the S-pole, and the control signal is output to the electromagnet driving unit 25. In this case, a magnetic force that is attraction is generated between the attachment magnet 11 and the electromagnet 21. As a result, the finger 2 of the user 1 is moved in a direction closer to the touch sensor 45.

In Fig. 4B, a control signal for causing a current to flow to the electromagnet 21 is generated so that the side of the touch sensor 45 of the electromagnet 21 is the N-pole, and the control signal is output to the electromagnet driving unit 25. In this case, a magnetic force that is repulsion is generated between the attachment magnet 11 and the electromagnet 21. As a result, the finger 2 of the user 1 is moved in a direction away from the touch sensor 45.

It should be noted that attraction and repulsion can be generated by setting the magnetic pole on the side of the touch sensor 45 of the electromagnet 21 to be the N-pole and the S-pole, respectively, in a case where the S-pole of the attachment magnet 11 faces the electromagnet 21.

In this manner, the body driving processing control unit 54 controls the electromagnet 21 so that a magnetic force that is attraction or repulsion is generated between the attachment magnet unit 10 (attachment magnet 11) and the electromagnet unit 20 (electromagnet 21). For example, the contact operation in which the user 1 touches the touch sensor 45 with the finger 2 can be promoted in generation of the attraction. Conversely, the contact operation can be suppressed in generation of the generating repulsion (repelling force).

As described above, the body driving processing control unit 54 uses the detection result of the spontaneous movement signal detection unit 53 as a trigger for the processing of moving the attachment magnet unit 10 (attachment magnet 11). For example, a timing (control timing T3) to control the attachment magnet unit 10 is set using the detection timing T0 of the spontaneous movement signal S1 as a basis. Alternatively, control, e.g., driving the attachment magnet unit 10 is performed in a case where the spontaneous movement signal S1 has not been detected by a set point of time. It will be described later in detail.

Moreover, the body driving processing control unit 54 acquires a reference signal for the movement of the user 1 of the body part and controls the attachment magnet unit 10 (attachment magnet 11) on the basis of the acquired reference signal. The reference signal is an external signal output from, for example, another computer such as the application controller 46 or a sensor for monitoring a situation or the like of the movement of the user 1.

Here, a signal indicating, for example, a timing of the contact operation by the finger 2 of the user 1 or necessity or unnecessity of the contact operation is read as the reference signal. Control parameters including timing and direction for driving the attachment magnet 11 are set on the basis of such a reference signal.

It should be noted that processing, e.g., driving the attachment magnet 11 may be performed with no reference signal.

In the present embodiment, the spontaneous movement signal detection unit 53 and the body driving processing control unit 54 cooperate to achieve the control unit.

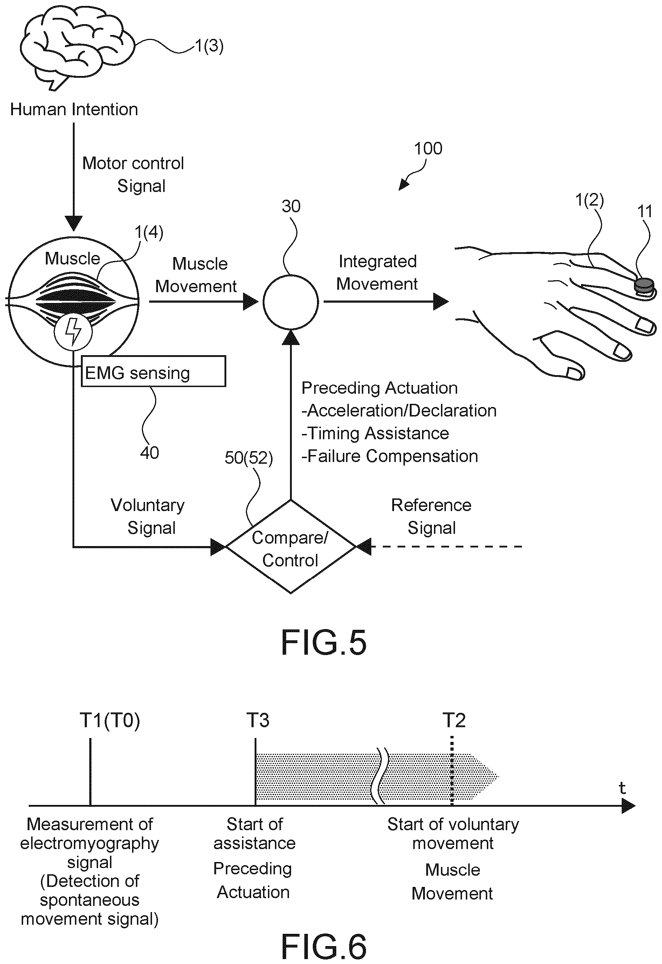

Fig. 5 is a schematic diagram for describing a basic operation of the body driving system 100. As shown in Fig. 5, a brain 3 of the user 1 generates a signal (motor control signal) for controlling a muscle 4. In a case where the user 1 performs a movement (voluntary movement) of moving the body part, the spontaneous movement signal S1 (voluntary Signal) is generated as the signal for controlling the muscle 4 and transmitted to the muscle 4. When the spontaneous movement signal S1 reaches the muscle 4, the muscle 4 starts to contract and a muscle movement occurs.

The body driving system 100 is a system that assists this muscle movement through the body driving unit 30 (e.g., the attachment magnet 11 and the electromagnet 21). Therefore, the movement of the body part (finger 2) of the user 1 is finally a movement (integrated movement) combining the movement by the body driving unit 30 and the muscle movement.

A final movement timing can be incorrect or a wrong movement can be assisted, for example, if the movement assistance is performed after the muscle movement of the user 1 occurs.

In view of this, in the present embodiment, the EMG sensor 40 detects a spontaneous movement signal S1 indicating a movement that the user 1 attempts to make. Then, the attachment magnet 11 and the electromagnet 21 perform movement assistance by electromagnetic driving, preceding the actual movement action.

As described above with reference to Fig. 4, the electromagnet 21 is, in the movement assistance by electromagnetic driving, activated to generate attraction or repelling force to the finger 2 of the user 1, to which the attachment magnet 11 has been attached, for moving the finger 2 of the user 1.

In general, a delay time from the driving start of the electromagnet 21 to the occurrence of the movement at the body part is shorter than a delay time from the detection of muscle force generation (spontaneous movement signal S1) at the EMG sensor 40 to the occurrence of the movement at the body part. Therefore, the movement of the finger 2 can be accelerated by, for example, about 20 msec by, for example, immediately driving the electromagnet 21 after detecting the spontaneous movement signal S1 in the movement of the finger 2.

Using such characteristics, the body driving system 100 starts the movement assistance to modify the body movement before (or at the same time as) the movement of the user 1 by referring to the spontaneous movement signal S1.

Fig. 6 is a schematic diagram for describing the start timing of the movement assistance.

Hereinafter, the timing when the EMG sensor 40 and the spontaneous movement signal detection unit 53 measure the EMG signal (action potential) will be referred to as a measurement timing T1.

Moreover, the timing when the finger 2 actually moves in accordance with the spontaneous movement signal S1 will be referred to as a movement timing T2. The movement timing T2 is set on the basis of, for example, characteristics of the finger 2 (e.g., a delay time of muscle force generation). For example, in a case where the spontaneous movement signal S1 has been detected at the measurement timing T1, the actual movement can be predicted to occur at the movement timing T2. In a case where the spontaneous movement signal S1 has not been detected at the measurement timing T1, the finger 2 of the user 1 can be predicted not to move at the movement timing T2. Thus, the movement timing T2 can be considered as a timing for the presence or absence of the voluntary movement, which is predicted at the measurement timing T1.

Moreover, the timing to start the movement assistance will be referred to as the control timing T3. The control timing T3 is the timing to control the attachment magnet unit 10 (attachment magnet 11), i.e., the timing to drive the electromagnet 21. The body driving processing control unit 54 sets the control timing T3.

The measurement timing T1, the movement timing T2, and the control timing T3 corresponds to a first point of time, a second point of time, and a third point of time, respectively.

In the present embodiment, the spontaneous movement signal detection unit 53 acquires an EMG signal (signal indicating the voluntary movement of the user 1) at the measurement timing T1.

At that time, a threshold determination as to the action potential of the EMG signal is performed and whether or not the spontaneous movement signal S1 has been generated is determined. For example, in a case where the action potential has changed and the spontaneous movement signal S1 has been detected, the measurement timing T1 equals the detection timing T0. In this case, the voluntary movement is started and the finger 2 of the user 1 actually moves at the movement timing T2 after the measurement timing T1.

The body driving processing control unit 54 sets the control timing T3 to perform movement assistance to drive the finger 2 of the user 1 before the movement timing T2. At that time, the contents of the movement assistance (e.g., the direction to drive the attachment magnet 11) are set to assist the movement predicted to occur at the movement timing T2.

That is, in the present embodiment, the body driving processing control unit 54 controls the attachment magnet 11 at the control timing T3 on the basis of the EMG signal of the user 1 at the measurement timing T1, the attachment magnet 11 being attached to the finger 2 of the user 1 and moving the finger 2 of the user 1, the control timing T3 being included in a period from the measurement timing T1 to the movement timing T2 when the movement of the finger 2 of the user 1 according to the EMG signal of the user 1 occurs. This enables the movement assistance to be performed at a suitable timing with no delay. It can suitably control the user's movement timing.

It should be noted that the control timing T3 may be arbitrarily set in a period from the measurement timing T1 to the movement timing T2, i.e., in a range of T1 < T3 < T2.

For example, the control timing T3 may be set to be substantially the same timing (T3 = T1) as the measurement timing T1. This enables, for example, the movement of the finger 2 of the user 1 to be sufficiently accelerated.

Moreover, for example, the control timing T3 may be set to be substantially the same timing (T3 = T2) as the movement timing T2. This enables, for example, the movement assistance to be performed without making the user 1 aware of it.

Various types of movement assistance can be achieved by performing preceding driving (preceding actuation) to move the attachment magnet 11 before the actual voluntary movement as described above.

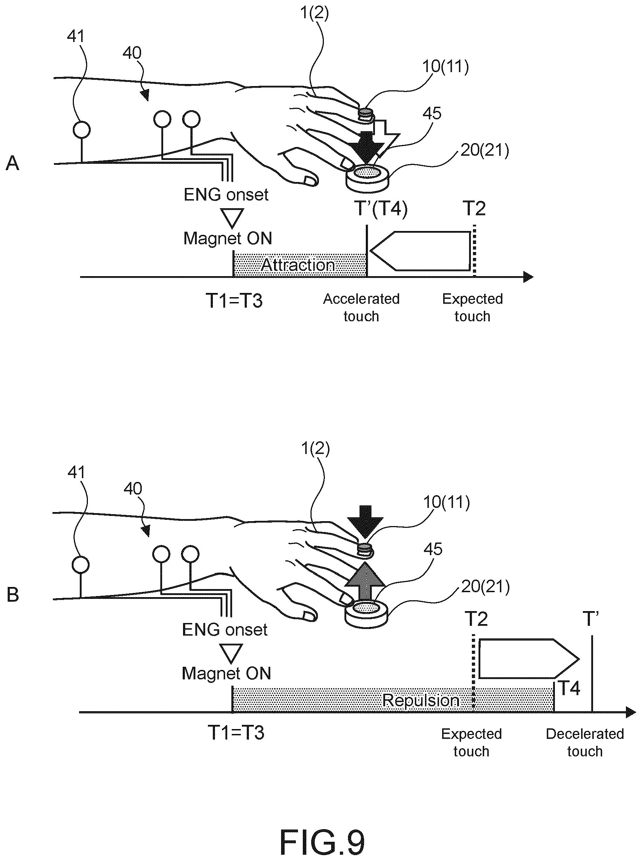

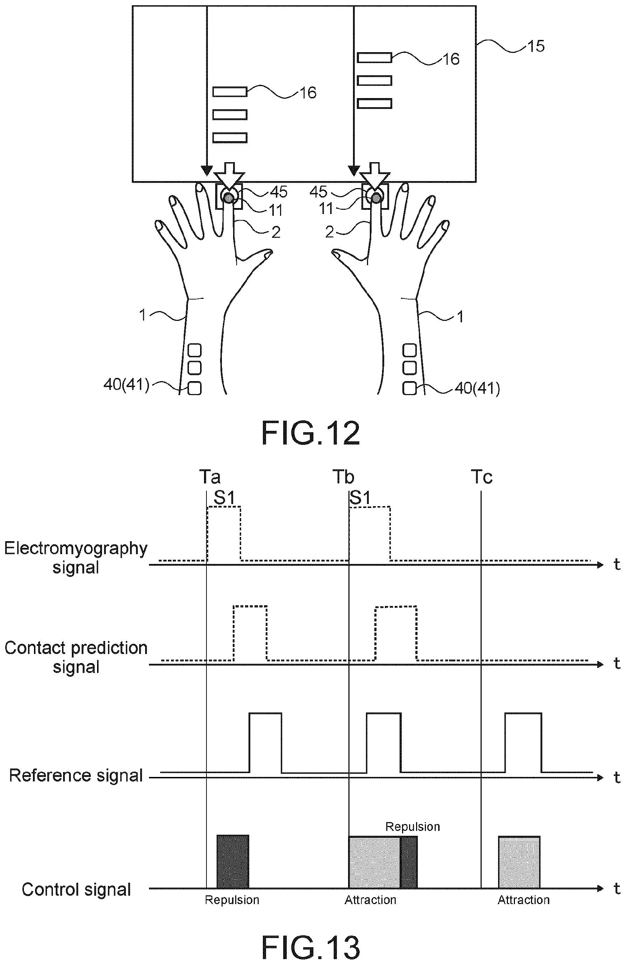

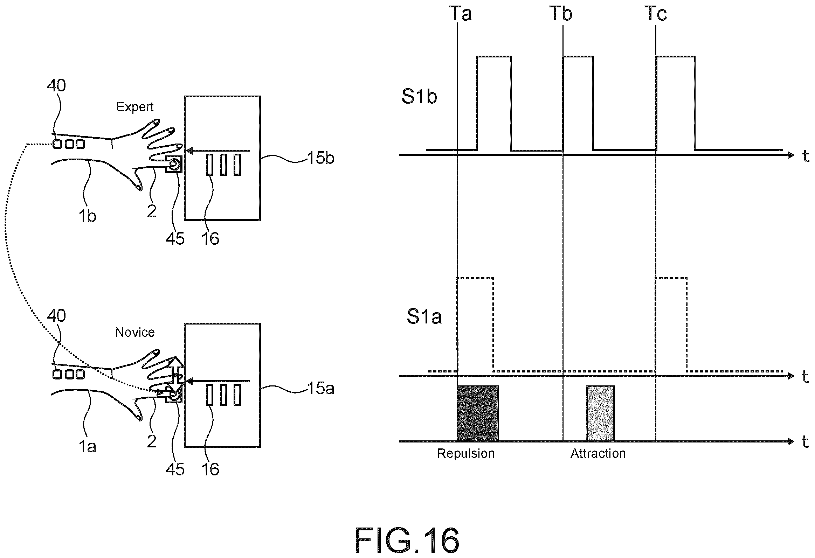

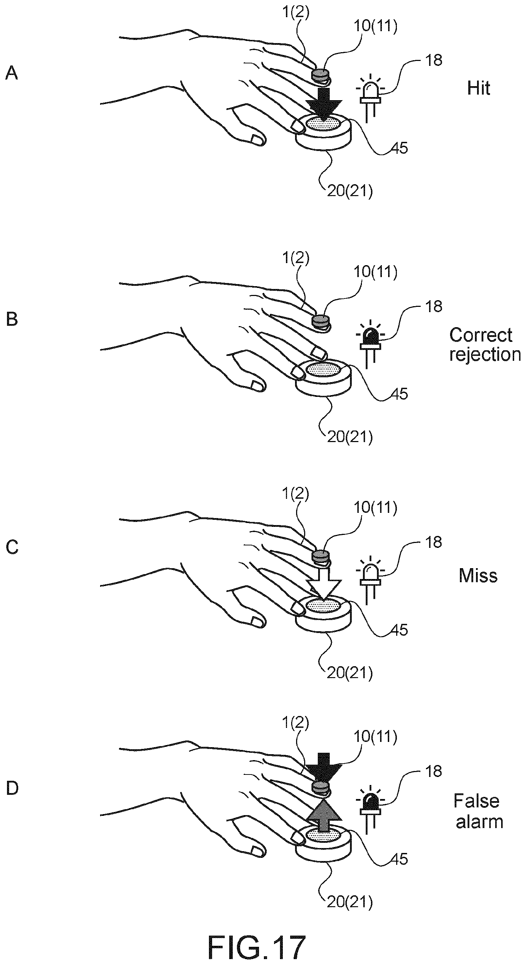

For example, the contact operation by the finger 2 of the user 1 can be accelerated or decelerated (see Fig. 9, etc.) Moreover, for example, the use of the reference signal enables control on the timing of the contact operation or the like (see Figs. 13 and 16, etc.) Moreover, for example, in a case where the user 1 erroneously performs the contact operation, movement assistance to correct the error can also be performed (see Fig. 17, etc.)

Hereinafter, each type of movement assistance will be described specifically.

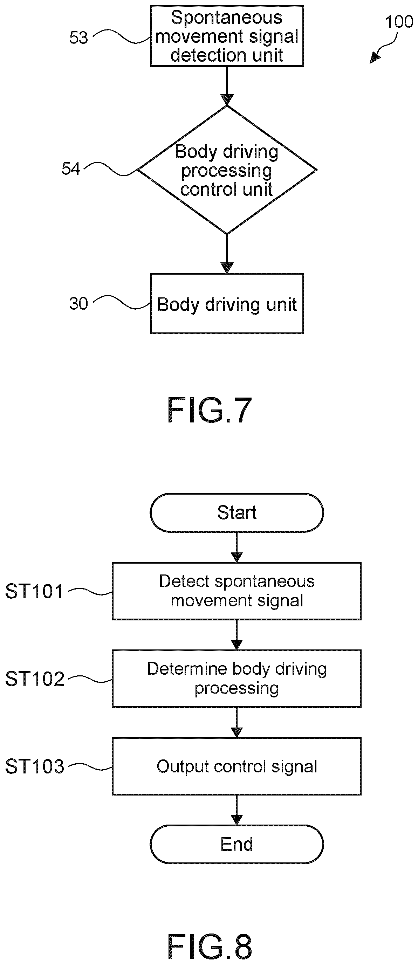

Fig. 7 is a block diagram showing a flow of movement assistance with no reference signal. Fig. 8 is a flowchart showing an example of the movement assistance with no reference signal.

In the movement assistance with no reference signal, the movement assistance is started in accordance with the preset control timing T3, for example, using the spontaneous movement signal S1 as a trigger.

In this case, as shown in Fig. 7, the detection result of the spontaneous movement signal detection unit 53 (presence or absence of the spontaneous movement signal S1) is input to the body driving processing control unit 54. Moreover, the body driving processing control unit 54 generates the control signal on the basis of the detection result. The body driving processing control unit 54 outputs the generated control signal to the body driving unit 30 (electromagnet driving unit 25).

Hereinafter, a flow of processing in the electromagnet controller 50 will be described with reference to Fig. 8.

The processing shown in Fig. 8 is loop processing repeatedly executed when performing the movement assistance.

First of all, the spontaneous movement signal detection unit 53 detects a spontaneous movement signal S1 from the EMG signal (Step 101). Specifically, the spontaneous movement signal detection unit 53 determines whether or not the value (action potential) of the EMG signal exceeds a predetermined threshold. For example, in a case where the value of the EMG signal exceeds the threshold, the spontaneous movement signal detection unit 53 determines that the spontaneous movement signal S1 has been generated. In a case where the value of the EMG signal has not exceeded the threshold, the spontaneous movement signal detection unit 53 determines that the spontaneous movement signal S1 has not been generated.

The result of determination processing is output as a trigger signal representing the presence or absence of the spontaneous movement signal S1, for example. Alternatively, the measurement timing T1 when the spontaneous movement signal S1 has been detected may be output as the detection timing T0.

Next, the body driving processing control unit 54 determines contents of the movement assistance (body driving processing) on the basis of the detection result of the spontaneous movement signal detection unit 53 (Step 102). Specifically, the body driving processing control unit 54 sets control parameters for driving the attachment magnet 11 (electromagnet 21).

For example, in a case where the spontaneous movement signal S1 has been detected, the body driving processing control unit 54 sets the control timing T3 to control the attachment magnet 11, using the detection timing T1 of the spontaneous movement signal S1 as a basis. Here, for example, the body driving processing control unit 54 sets a point of time after a predetermined waiting time has elapsed from the detection timing T1 as the control timing T3. The body driving processing control unit 54 sets the predetermined waiting time to zero in a case where of immediately driving the attachment magnet 11, for example. It should be noted that the predetermined waiting time may be set as appropriate within a range equal to or shorter than the delay time of muscle force generation.

Moreover, the body driving processing control unit 54 sets direction and period to drive the attachment magnet 11. Here, the body driving processing control unit 54 sets the direction and the period to drive the attachment magnet 11 to be preset direction and period.

It should be noted that the subsequent processing is not executed in a case where the spontaneous movement signal S1 has not been detected.

Next, the body driving processing control unit 54 generates a control signal for the electromagnet 21 in accordance with the set control parameters and outputs the control signal to the electromagnet driving unit 25 (Step 103). The control signal is, for example, a pulse signal whose voltage rises at the control timing T3. The positive and negative value of the voltage of this pulse signal corresponds to an orientation of the magnetic pole generated in the electromagnet 21. Moreover, the width of the pulse signal corresponds to a period to generate a magnetic force, i.e., a period to drive the attachment magnet 11.

It should be noted that the waveform of the pulse signal may be set as appropriate in accordance with the kind and the like of the movement assistance. For example, setting a rectangular pulse signal can increase the velocity of driving the attachment magnet 11. Moreover, for example, a pulse signal that rises with a constant gradient may be set. In this case, the force applied to the attachment magnet 11 can be gradually changed. It can achieve natural movement assistance.

Fig. 9 is a schematic diagram showing an example of movement assistance to accelerate or decelerate the movement of the finger 2. Here, processing of accelerating or decelerating the contact operation by the finger 2 of the user 1 will be described as an example of the movement assistance with no reference signal. Moreover, the waiting time for the control timing T3 is set to zero and the attachment magnet 11 (electromagnet 21) is driven immediately after detecting the spontaneous movement signal S1.

In Fig. 9A, the movement assistance to accelerate the contact operation is executed. In this case, the electromagnet 21 is driven to move the attachment magnet 11 in the same direction as a movement direction (black arrow in the figure) in which the user 1 moves the finger 2 for performing the contact operation. Here, attraction (white arrow in the figure) between the attachment magnet 11 and the electromagnet 21 is generated and the attachment magnet 11 is driven in a direction closer to the electromagnet 21.

Accordingly, the finger 2 of the user 1 is moved toward the touch sensor 45 earlier than the movement based on the voluntary movement. As a result, a contact timing T' when the finger 2 of the user 1 touches the touch sensor 45 is earlier than the movement timing T2 in a case where no movement assistance is performed.

It should be noted that in Fig. 9A, driving of the electromagnet 21 is stopped and the attraction is cut when the finger 2 of the user 1 touches the touch sensor 45. This enables the next contact operation to be smoothly performed. Hereinafter, the timing to terminate driving of the electromagnet 21, i.e., the timing to terminate the control on the attachment magnet 11 will be referred to as an end timing T4. In the present embodiment, the end timing T4 corresponds to a fourth point of time.

In Fig. 9B, movement assistance to decelerate the contact operation is executed. In this case, the electromagnet 21 is driven to move the attachment magnet 11 in a direction opposite to the movement direction in which the user 1 moves the finger 2. Here, repulsion (gray arrow in the figure) is generated between the attachment magnet 11 and the electromagnet 21 and the attachment magnet 11 is driven in the direction away from the electromagnet 21.

At that time, the period to act the repulsion on the attachment magnet 11 (repulsion end timing T4) is set to be longer than the movement timing T2. That is, the repulsion is applied to the attachment magnet 11 also after the movement timing T2.

Accordingly, a force to move away from the touch sensor 45 acts on the finger 2 of the user 1 (attachment magnet 11) also after the movement by the voluntary movement occurs at the movement timing T2. As a result, the contact timing T' when the finger 2 of the user 1 touches the touch sensor 45 is later than the movement timing T2 in a case where no movement assistance is performed.

As described above, in the present embodiment, in a case where the spontaneous movement signal S1 has been detected, the attachment magnet 11 is controlled to accelerate or decelerate the movement (contact operation) of the finger 2 of the user 1 using the movement timing T2 as a basis.

This is movement assistance to adjust the timing when the user 1 performs the contact operation with the finger 2 to be earlier or later than the original movement timing 3. Combining such processing as appropriate allows accurate control on the timing of the contact operation.

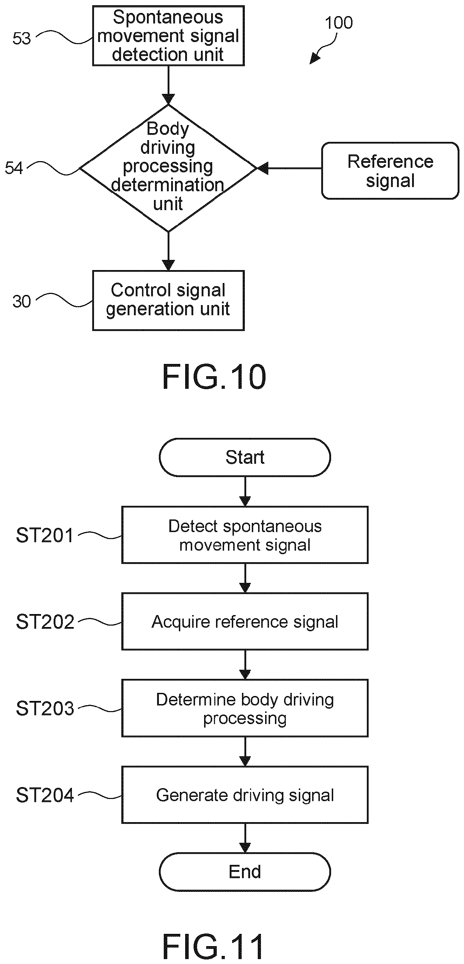

Fig. 10 is a block diagram showing a flow of movement assistance with the reference signal. Fig. 11 is a flowchart showing an example of the movement assistance with the reference signal.

In the movement assistance with the reference signal, the movement assistance is set by comparing, for example, the detection timing T0 of the spontaneous movement signal S1 and the presence or absence of the spontaneous movement signal S1 with the reference signal.

In this case, as shown in Fig. 11, the detection result of the spontaneous movement signal detection unit 53 and the reference signal are input to the body driving processing control unit 54. The body driving processing control unit 54 compares the detection result of the spontaneous movement signal detection unit 53 with the reference signal and generates a control signal on the basis of the comparison result. The generated control signal is output to the body driving unit 30 (electromagnet driving unit 25).

Hereinafter, a flow of processing in the electromagnet controller 50 will be described with reference to Fig. 11.

The processing shown in Fig. 11 is loop processing repeatedly executed when performing the movement assistance.

First of all, the spontaneous movement signal detection unit 53 detects a spontaneous movement signal S1 from the EMG signal (Step 201). This processing is processing similar to that of Step 101 shown in Fig. 9. The spontaneous movement signal detection unit 53 outputs a trigger signal representing the presence or absence of the spontaneous movement signal S1 and the detection timing T0 of the spontaneous movement signal S1.

Next, the body driving processing control unit 54 acquires a reference signal (Step 202). The reference signal is, for example, output from a computer different from the electromagnet control unit 52 of the electromagnet controller 50 and read by the body driving processing control unit 54.

Next, the body driving processing control unit 54 determines contents of the movement assistance (body driving processing) on the basis of the detection result of the spontaneous movement signal detection unit 53 and the reference signal (Step 203).

For example, the reference signal is a signal indicating the timing of the movement (contact operation) of the finger 2 of the user 1. In this case, the control parameters to accelerate or decelerate the movement of the finger 2 of the user 1 are set so that the user 1 performs the contact operation with the finger 2 in accordance with the timing specified by the reference signal.

Moreover, for example, the reference signal is a signal for specifying necessity or unnecessity of the contact operation that the user 1 performs by moving the finger 2 of the user 1. In this case, the reference signal is a signal indicating a situation where the user 1 should perform the contact operation by moving his or her finger 2 or a situation where the user 1 should not perform the contact operation by moving his or her finger 2. In the present embodiment, the contact operation corresponds to a predetermined movement.

The body driving processing control unit 54 compares the necessity or unnecessity of the contact operation indicated by the reference signal with the presence or absence of the spontaneous movement signal S1 and controls the attachment magnet 11 on the basis of this comparison result. Specifically, the body driving processing control unit 54 sets movement assistance (control parameters) to drive the attachment magnet 11 to promote or suppress the contact operation.

Next, the body driving processing control unit 54 generates a control signal for the electromagnet 21 in accordance with the set control parameters and outputs the control signal to the electromagnet driving unit 25 (Step 204). This processing is processing similar to that of Step 103 shown in Fig. 9.

This enables, for example, the user 1 to perform the contact operation at the timing specified by the reference signal and avoid an error in the movement of the user 1 and the like.

Fig. 12 is a schematic diagram showing an example of the movement assistance to control the timing of the contact operation. Here, the processing of controlling the timing of the contact operation by the finger 2 of the user 1 will be described as an example of the movement assistance with the reference signal.

Fig. 12 schematically shows a state in which the user 1 plays a rhythm game using the index fingers of the both hands. The rhythm game is an application executed by the application controller 46.

Here, a plurality of rhythm marks 16 for the right and left hands is displayed on a display 15. These rhythm marks 16 scroll down on the display 15. The rhythm game is a game where a player plays rhythm by touching the touch sensor 45 with the finger in accordance with a timing when each of the rhythm marks 16 reaches a predetermined line.

In the example shown in Fig. 12, a signal indicating the timing when the rhythm mark 16 reaches a predetermined line, i.e., a signal indicating a suitable timing of the contact operation is used as a reference signal.

In this manner, the movement of the finger 2 of the user 1 is a movement for performing an input operation for a predetermined application (rhythm game). Moreover, the reference signal is a signal indicating the timing of the input operation.

This reference signal is generated by the application controller 46 and read by the body driving processing control unit 54. It should be noted that the reference signal can be considered as a signal indicating the situation where the contact operation should be performed by showing the timing of the contact operation.

Fig. 13 is a graph showing control contents of the movement assistance shown in Fig. 12. Fig. 13 shows a schematic graph showing the EMG signal of the finger 2 of the user 1, a contact prediction signal predicted from the EMG signal, a reference signal for the contact operation, and a control signal for controlling the electromagnet 21.

It should be noted that Fig. 13 schematically shows the spontaneous movement signal S1 in the EMG signal as a rectangular pulse. Moreover, the contact prediction signal is, for example, a signal predicted from the start and end timings of the spontaneous movement signal S1, and represents a contact operation performed in a case where no movement assistance is performed.

In Fig. 13, the spontaneous movement signal S1 is detected at a point of time Ta. In this case, the time (movement timing T2) when the user 1 is predicted to touch the touch sensor 45 with the finger 2 is earlier than rising of the reference signal. In this case, the contact operation of the user 1 can be performed earlier than the timing specified by the reference signal.

Therefore, the movement assistance to decelerate the contact operation is executed so that the contact operation of the user 1 occurs at the timing specified by the reference signal. Specifically, the control signal (pulse signal) to generate repulsion in the attachment magnet 11 is generated. Pulse signals of the repulsion are shown as the darker gray regions.

The timing (control timing T3) to turn on the pulse signal of the repulsion is set to precede rising (movement timing T2) of the contact prediction signal. This enables the contact operation of the user 1 to be reliably decelerated.

Moreover, the timing (end timing T4) to turn off the pulse signal of the repulsion is set to precede the timing when the reference signal rises. This enables the user 1 to touch the touch sensor 45 with the finger 2 at the timing indicated by the reference signal.

Moreover, in Fig. 13, the spontaneous movement signal S1 is detected also at a point of time Tb. In this case, the time (movement timing T2) when the user 1 is predicted to touch the touch sensor 45 with the finger 2 is later than rising of the reference signal. In this case, the contact operation of the user 1 can be performed later than the timing specified by the reference signal.

Therefore, the movement assistance to accelerate the contact operation is executed so that the contact operation of the user 1 occurs at the timing specified by the reference signal. Specifically, the control signal (pulse signal) to generate attraction for the attachment magnet 11 is generated. Pulse signals of the attraction are shown as the lighter gray regions.

The timing (control timing T3) to turn on the pulse signal of the attraction is set on the basis of the timing when the reference signal rises. Here, the timing when the reference signal rises is substantially close to the point of time Tb, and therefore the pulse signal of the attraction is set to be on at substantially the same time as the point of time Tb. It should be noted that in a case where it takes a time until the timing when the reference signal rises comes, for example, the timing to turn on the pulse signal of the attraction is set so that the contact of the finger 2 of the user 1 occurs at that timing. This allows accurate control on the timing of the contact operation of the user 1.

Moreover, the timing (end timing T4) to turn off the pulse signal of the attraction is set to be the timing when the reference signal falls. This enables the user 1 to smoothly move away the finger with which the user has touched the touch sensor 45.

Moreover, the period of the contact operation predicted from the spontaneous movement signal S1 detected at the point of time Tb is longer than the period specified by the reference signal. Thus, the contact operation can be continued also after the period specified by the reference signal. Therefore, in the example shown in Fig. 13, the processing of generating repulsion is executed at the timing when the reference signal falls. This enables the user 1 to move the finger 2 away from the touch sensor 45. It enables the user 1 to smoothly perform the next contact operation.

In this manner, the body driving processing control unit 54 sets, on the basis of the timing of the movement of the finger 2 of the user 1 indicated by the reference signal, at least one of the control timing T3 to control the attachment magnet 11 or the end timing T4 to terminate the control on the attachment magnet 11.

Moreover, as shown in Fig. 13, the spontaneous movement signal S1 is not detected at a point of time Tc. On the other hand, the pulse of the reference signal is found at a point of time (i.e., the movement timing T2 using the point of time Tc as a basis) delayed from the point of time Tc by the delay time of muscle force generation. It corresponds to a case where the user 1 fails to perform the next contact operation by mistake at the point of time Tc.

In this manner, in a case where the spontaneous movement signal S1 to make the contact operation has not been detected in a situation where the contact operation is necessary, the attachment magnet 11 is controlled so that the user 1 performs the contact operation with the finger 2.

Here, the control signal (pulse signal) to generate attraction is generated so as to perform the contact operation. In this case, the timing to turn on the pulse signal of the attraction is set so that the contact of the finger 2 of the user 1 occurs in time on the basis of the timing when the reference signal rises. Moreover, the timing to turn off the pulse signal of the attraction is set to be the timing when the reference signal falls.

This enables the contact operation to be performed at an appropriate timing even with respect to the rhythm mark 16 or the like that the user 1 has not reacted.

As described above, in the present embodiment, the detection result of the spontaneous movement signal S1 is compared with the timing (reference signal) when that movement should be performed. Then, the movement velocity and the movement timing of the body of the user 1 can be controlled at a high time-accuracy by combining the movement assistance to accelerate/decelerate the contact operation on the basis of the comparison result.

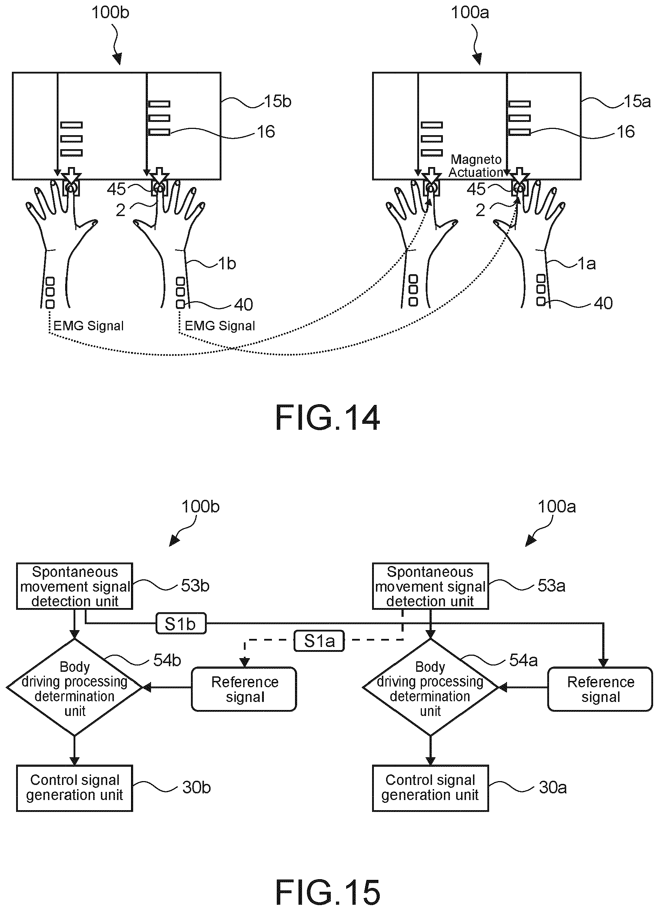

Fig. 14 is a schematic diagram showing an example of the movement assistance in conjunction with the other user. Fig. 15 is a block diagram showing a flow of the movement assistance shown in Fig. 14. Here, movement assistance to coordinate/synchronize a contact operation of a user 1a and a contact operation of another user 1b will be described. In this movement assistance, a spontaneous movement signal detected from the signal indicating the voluntary movement (EMG signal of the other user 1b) of the other user 1b different from the user 1a is used as a reference signal.

Hereinafter, the spontaneous movement signals of the users 1a and 1b will be referred to as S1a and S1b, respectively. S1b of them is a reference signal for performing the movement assistance of the user 1a.

Fig. 14 schematically shows a state in which the user 1a and the user 1b play a rhythm game together. Game screens operated by the user 1a and the user 1b are displayed on right and left displays 15a and 15b in the figure. It should be noted that the illustration of the attachment magnet 11 is omitted from Fig. 14.

Moreover, body driving systems 100a and 100b that perform movement assistance for the users 1a and 1b are connected to each other in this movement assistance as shown in Fig. 15. Specifically, the body driving system 100b (spontaneous movement signal detection unit 53b) of the user 1b sends the spontaneous movement signal S1b detected from the EMG signal of the user 1b to the body driving system 100a (body driving processing control unit 54a) of the user 1a. Control on the timing of the contact operation of the user 1a or control to make or suppress the contact operation is performed on the basis of the spontaneous movement signal S1b of the user 1b. This allows coordination/synchronization of movements of two people.

Fig. 16 is a schematic diagram for describing the control contents of the movement assistance shown in Fig. 14. Here, training assuming the same movement is performed presenting rhythm marks 16 to the two people, the users 1a and 1b, at the same timing. In this training, for example, the user 1a who is a novice learns the movement of the user 1b who is an expert.

On the upper side of Fig. 16, the spontaneous movement signal S1b detected from the EMG signal of the user 1b is shown as the solid-line graph. Moreover, a spontaneous movement signal S1a detected from the EMG signal of the user 1a is shown as the dotted-line graph on the lower side of Fig. 16.

In Fig. 16, the spontaneous movement signal S1a of the user 1a is detected at the point of time Ta. At this time, the spontaneous movement signal S1b of the user 1b has not yet been detected. In this case, the contact operation of the user 1a can be performed earlier than the contact operation of the user 1b.

Therefore, the movement assistance to decelerate the contact operation is executed so that the contact operation of the user 1a occurs at the timing specified by the spontaneous movement signal S1b of the user 1b. Specifically, the control signal (pulse signal) to generate repulsion in the attachment magnet 11 is generated.

It should be noted that the timing specified by the spontaneous movement signal S1b is a timing when the user 1b is predicted to touch the touch sensor 45 with the finger 2 in a voluntary movement that occurs in accordance with S1b, for example. This is the movement timing T2 for the user 1b and is calculated on the basis of S1b detected immediately after the point of time Ta. Here, the pulse signal of the repulsion is set to be off right before the user 1b touches the touch sensor 45 with the finger 2.

Moreover, in Fig. 16, the spontaneous movement signal S1b of the user 1b is detected at the point of time Tb. On the other hand, the spontaneous movement signal S1a of the user 1a is not detected at the point of time Tb. In this case, the contact operation of the user 1a can be delayed from the contact operation of the user 1b or the contact operation of the user 1a can be missed.

In this manner, in a case where the spontaneous movement signal S1a to make the contact operation has not been detected in a situation where the contact operation is necessary, the attachment magnet 11 is controlled so that the user 1a performs the contact operation with the finger 2.

Here, a control signal (pulse signal) to generate attraction is generated so as to perform the contact operation. In this case, the timing to turn on the pulse signal of the attraction is set right before the timing when the contact operation of the user 1b is performed, for example. Moreover, the timing to turn off the pulse signal of the attraction is set at the timing when the contact operation of the user 1b ends.

This enables the contact operation of the user 1a to be made (or accelerated) in accordance with the movement of the user 1b.

Moreover, in Fig. 16, the spontaneous movement signals S1a and S1b of the user 1a and the user 1b are both detected at the point of time Tc. In this case, considering that the timings of the contact operations of the user 1a and the user 1b are substantially the same, the electromagnet 21 (attachment magnet 11) is not driven generating no control signal.

This enables the contact operation of the user 1a to be controlled to be synchronized with the contact operation of the user 1b at a high time-accuracy on the basis of on the contact operation of the user 1b. This enables the novice user 1a to directly experience and learn the contact operation of the expert user 1b.

It should be noted that for example, the contact prediction signal or the like of the user 1b calculated on the basis of the spontaneous movement signal S1b may be used as a reference signal rather than using the spontaneous movement signal S1b of the user 1b as the reference signal as it is.

The above-mentioned training is processing of synchronizing the movement of the user 1a with the movement of the user 1b. However, the present technology is not limited thereto. For example, movement assistance to synchronize the movements of the users 1a and 1b with each other may be achieved. In this case, the spontaneous movement signal S1a detected from the EMG signal of the user 1a is sent to the body driving system 100b (body driving processing control unit 54b) of the user 1b as shown in the dotted line in Fig. 15. This allows synchronization of their movements.

Moreover, the movement of the user 1b in a situation where the spontaneous movement signal S1a of the user 1a has been detected is suppressed in a case where the user 1a and the user 1b should perform exclusive movements. Conversely, the movement of the user 1a is suppressed in a case where the spontaneous movement signal of the user 1b has been detected. This allows assistance for coordinated movements to perform movements opposite to each other at a high time-accuracy. It should be noted that inverting values of reference signals of the users 1a and 1b may achieve exclusive synchronization.

Fig. 17 is a schematic diagram showing an example of movement assistance for a movement involving a cognitive judgement. Here, movement assistance to ensure a perceptual or cognitive error of the user 1 in a task/movement in which the user 1 needs a cognitive judgement will be described.

The task that needs the cognitive judgement is, for example, a task to recognize a situation on the basis of a signal color and determine whether or not to perform a movement. In this case, a perceptual error, e.g., mistaking the signal color and a cognitive error, e.g., misreading a situation associated with the signal color can occur.

In order to avoid such an error, a signal indicating necessity or unnecessity of the contact operation (signal indicating a situation where the contact operation should be performed or a situation where the contact operation should not be performed) is used here as a reference signal.

This enables the body driving processing control unit 54 to determine whether or not to execute a movement that occurs in accordance with the spontaneous movement signal S1 of the user 1. Moreover, in a case of changing a movement that occurs in accordance with the spontaneous movement signal S1, movement assistance for making a necessary change/intervention to the movement of the user 1 is set and the body driving processing control unit 54 assists the movement of the user 1.

In Fig. 17, the user 1 performs a task of touching the touch sensor 45 in accordance with the color of a sign 18. Here, the blue color (white color in Fig. 17) of the sign 18 represents a go-sign. The go-sign is a sign indicating a situation where the user 1 should touch the touch sensor 45. Moreover, the red color (black color in Fig. 17) of the sign 18 represents a not-go-sign. The not-go-sign is a sign indicating a situation where the user 1 should not touch the touch sensor 45.

The signals indicating the go-sign and the not-go-sign are used as reference signals.

In Fig. 17A, a spontaneous movement signal S1 to make the contact operation of the user 1 is detected in a situation where the go-sign has been presented. In this case, the task result can be predicted to be a suitable contact operation (hit) before the finger 2 of the user 1 actually touches the touch sensor 45. Therefore, no movement assistance to drive the attachment magnet 11 (electromagnet 21) is performed.

In Fig. 17B, the spontaneous movement signal S1 to make the contact operation of the user 1 is not detected in a situation where the not-go-sign has been presented. In this case, considering that the finger 2 of the user 1 will not touch the touch sensor 45, the task result can be predicted to be suitable avoidance (correct rejection) of the contact operation. Therefore, no movement assistance to drive the attachment magnet 11 (electromagnet 21) is performed also in Fig. 17B.

In Fig. 17C, the spontaneous movement signal S1 to make the contact operation of the user 1 is not detected in a situation where the go-sign has been presented. In this case, considering that the finger 2 of the user 1 will not touch the touch sensor 45, the task result can be predicted to be non-execution (miss) of the contact operation.

In this manner, in a case where the spontaneous movement signal S1 to make the contact operation has not been detected in a situation where the contact operation is necessary, the attachment magnet 11 is controlled so that the user 1 performs the contact operation with the finger 2. Here, the electromagnet 21 is driven to apply attraction (white arrow in the figure) on the attachment magnet 11.

In Fig. 17D, the spontaneous movement signal S1 to make the contact operation of the user 1 is detected in a situation where the not-go-sign has been presented. In this case, the task result can be predicted to be an unsuitable contact operation (false alarm) before the finger 2 of the user 1 actually touches the touch sensor 45.

In this manner, in a case where the spontaneous movement signal S1 to make the contact operation has been detected in a situation where the contact operation is unnecessary, the attachment magnet 11 is controlled so that the finger 2 of the user 1 does not perform the contact operation. Here, the electromagnet 21 is driven to apply repulsion (gray color arrow in the figure) on the attachment magnet 11.

As shown in Fig. 17C and 17D, the user 1 makes an action with a delay irrespective of the go-sign or makes an action irrespective of the not-go-sign. In this regards, with the present technology, the action can be made in time by executing the movement assistance in a case where the user 1 has moved with a delay irrespective of the go-sign. Moreover, movement assistance to suppress the movement before the movement occurs can be performed because the spontaneous movement signal S1 can be detected before the movement occurs in a case where the user 1 attempts to move irrespective of the not-go-sign.

This allows avoidance in advance of a cognitive or perceptual error of the user 1. It can sufficiently enhance the accuracy of the contact operation of the user 1 or the like.

As described above, the electromagnet control unit 52 according to the present embodiment assists the movement of the finger 2 (body part) of the user 1 that occurs at the movement timing T2 in accordance with the EMG signal of the user 1 at the measurement timing T1 by moving the attachment magnet unit 10 attached to the finger 2. This attachment magnet unit 10 is driven at the control timing T3 included in the period from the measurement timing T1 to the movement timing T2. This enables assistance to start preceding the movement of the body part of the user. It can suitably control the user's movement timing.

One of methods of assisting a human movement is a method of adding assistance or correction to a human movement after detecting the movement. In this method, a time delay due to signal detection or processing sometimes occurs. Moreover, a delay due to a mechanical movement sometimes occurs in a case of using a method of using a mechanical mechanism (e.g., wearable exoskeleton) as a method of driving a human body. Moreover, a delay also occurs between excitation of the muscle and actual movement of the body in a case of using an electrical muscle stimulation (EMS) for stimulating a muscle itself to excite the muscle.

Such a delay of the signal processing or a delay associated with the driving method makes it difficult to perform a movement in a suitable timing or to suppress a movement after the movement starts.

In the present embodiment, the control timing T3 to drive the finger 2 is set to precede the movement timing T2 when the movement of the finger 2 of the user 1 occurs. This enables the finger 2 of the user 1 to be driven preceding the movement of the user 1 that occurs due to the signal indicating the voluntary movement. This allows sufficient avoidance of a time delay due to signal detection or processing and appropriate control on the movement timing of the user 1.

Moreover, in the present embodiment, an electromagnetic force-based drive mechanism (attachment magnet unit 10 and electromagnet unit 20) is used as the body driving unit 30 that moves the finger 2 of the user 1. Therefore, the body driving unit 30 has no delay of a mechanical driving time and no delay due to muscle force generation. This enables the finger 2 of the user 1 to be driven at a high temporal resolution, for example. It allows sufficiently highly accurate control on the movement timing of the user 1.

### <Second Embodiment>

A body driving system according to a second embodiment of the present technology will be described. Hereinafter, descriptions of portions similar to the configurations and actions in the body driving system 100 described in the above embodiment will be omitted or simplified.

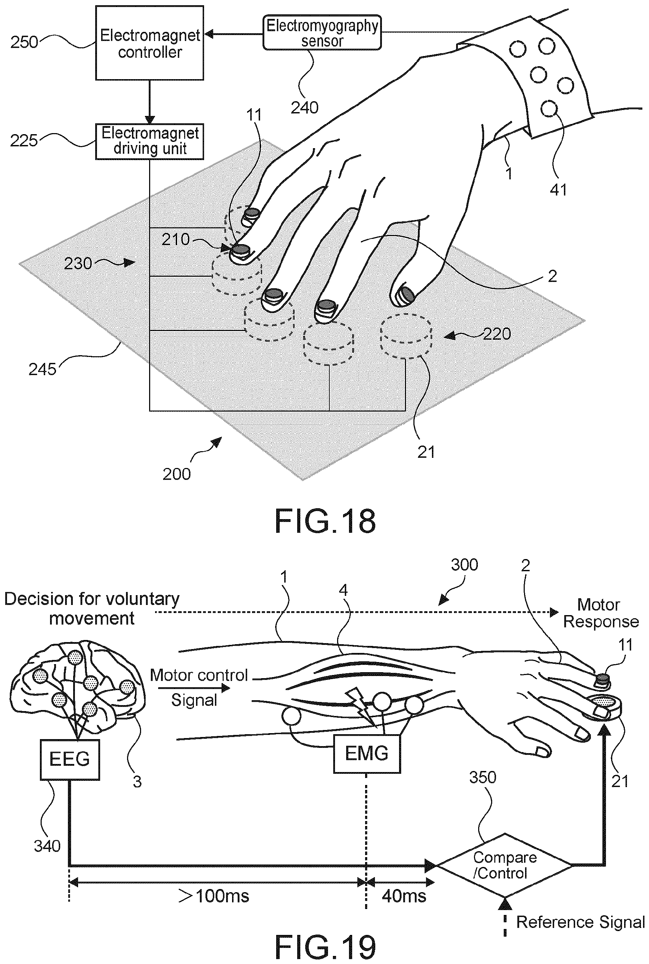

Fig. 18 is a schematic diagram showing a configuration example of a body driving system 200 according to the second embodiment. The body driving system 200 is a system that performs movement assistance for the contact operation for each of five fingers 2 of the hand of the user 1.

The body driving system 200 includes a body driving unit 230, an EMG sensor 240, a screen interface 245, and an electromagnet controller 250.

The body driving unit 230 includes an attachment magnet unit 210, an electromagnet unit 220, and an electromagnet driving unit 225.

The attachment magnet unit 210 includes an attachment magnet 11 attached to each finger 2 of the user 1.

The electromagnet unit 220 includes at least one electromagnet 21 that generates a magnetic field that acts on the attachment magnet 11. In the example shown in Fig. 18, the attachment magnet 11 is attached to each of the five fingers 2 of the right hand of the user 1 and is provided in five electromagnets 21 corresponding to each finger 2 (each attachment magnet 11). In this manner, the electromagnet unit 220 includes at least one electromagnet 21 disposed in the screen interface 245 that corresponds to the finger 2 of the user 1.

The electromagnet driving unit 225 individually drives each electromagnet 21 on the basis of a control signal output from the electromagnet controller 250.

The EMG sensor 240 individually detects excitation of a muscle as an observation target through a plurality of surface electrodes 41. The surface electrodes 41 are respectively disposed at positions where they can detect EMG signals of muscles that move the respective fingers 2 of the user 1. Fig. 18 schematically shows a band-type attachment tool including the plurality of surface electrodes 41.

The EMG sensor 240 detects an action potential of the muscle measured by each surface electrode 41 and outputs the action potential to the electromagnet controller 250 as the EMG signal for each finger 2.

The screen interface 245 is a display including a touch screen that the user 1 touches and operates with the finger 2. The above-mentioned electromagnet unit 220 is provided in the screen interface 245. More specifically, the plurality of electromagnets 21 corresponding to the respective fingers 2 is disposed at the back of a movement surface of the screen interface 245 (surface touched by the finger 2 of the user 1).

In the present embodiment, the electromagnet 21 is driven when the user 1 performs a contact operation by moving the finger 2 with respect to the screen interface 245 as an input apparatus.

The electromagnet controller 250 individually drives the attachment magnet 11 attached to each finger 2 by individually controlling each electromagnet 21. For example, the electromagnet controller 250 detects the spontaneous movement signal S1 of the finger 2 of the user 1 from the EMG signal measured by the EMG sensor 240 and generates a control signal for driving the corresponding electromagnet 21 on the basis of the detection result. These processes are independently executed for each finger 2.

This allows movement assistance, e.g., accelerating/decelerating each of the five fingers 2 of the user 1. Moreover, the input operation with respect to the screen interface 245 can be controlled at a high time-accuracy. It should be noted that the electromagnet unit 220 may be used for movement assistance for keyboard and touch panel operations, for example. This can achieve an accurate and high-speed input operation or the like.

### <Third Embodiment>

Fig. 19 is a schematic diagram showing a configuration example of a body driving system according to a third embodiment. A body driving system 300 uses an electroencephalography signal detected in the brain of the user 1 as a signal indicating a voluntary movement of the user 1 instead of the above-mentioned EMG signal.

Fig. 19 schematically shows a flow of a signal (spontaneous movement signal) for transmitting a command of the voluntary movement during the period from the time when the user 1 decides to perform the voluntary movement to the time when the movement actually occurs. The flow of the spontaneous movement signal is basically the same as the one described above with reference to Fig. 5.

The body driving system 300 has an electroencephalography (EEG) sensor 340 for measuring an EEG of the user 1. The EEG sensor 340 is, for example, an electroencephalograph with a non-invasive electrode unit that the user 1 puts on the head for use. Fig. 19 schematically shows the electrode unit of the EEG sensor 340. It should be noted that a sensor including an invasive electrode unit, a semi-invasive electrode unit, or the like may be used as the EEG sensor 340.

The EEG sensor 340 measures an EEG signal and inputs it to an electromagnet controller 350. The electromagnet controller 350 detects, for example, a signal (spontaneous movement signal) of a voluntary movement to move the finger 2 from the EEG signal and drives the electromagnet 21 on the basis of the detection result.

For example, in a case where a voluntary movement to move the finger 2 occurs, the spontaneous movement signal detected from the EMG signal of the muscle of the finger 2 is a signal preceding a timing when the finger 2 actually moves by about 40 msec.

In this regards, it is assumed that the spontaneous movement signal for making a voluntary movement to move the finger 2 is detected from the EEG signal. In this case, since the time needed for a signal to pass through the spinal cord and the like is omitted, the occurrence of the voluntary movement can be detected preceding the EMG signal by about 100 msec, for example.

Therefore, the movement of the user 1 can be reliably assisted by detecting a spontaneous movement signal through the EEG sensor 340.

Moreover, a signal (spinal cord signal) detected in the spinal cord located at a certain point on a path connecting the brain 3 and the muscle 4 may be used as the signal indicating the voluntary movement of the user 1. Also in this case, the occurrence of the voluntary movement can be detected at a timing earlier than the EMG signal.

In addition, any signal indicative of the occurrence of the voluntary movement of the user 1 may be used.

### <Other Embodiments>

The present technology is not limited to the above-mentioned embodiments, and various other embodiments can be achieved.

Hereinabove, the method of setting the timing (control timing T3) to drive the electromagnet in accordance with the timing indicated by the spontaneous movement signal S1 or the reference signal has been described. The strength of driving force (attraction and repulsion of the electromagnet) to drive the attachment magnet may be set when setting the control timing T3.

For example, increasing the strength of the attraction speeds up the attraction of the finger of the user (attachment magnet). Moreover, for example, increasing the strength of the repulsion can also delay the timing when the user (attachment magnet) touches the touch sensor with the finger. In this way, adjusting the strength of the attraction or repulsion can adjust the speed or the like of the contact operation.

Moreover, at least one of the control timing T3 or the end timing T4 may be set in accordance with a driving force to drive the attachment magnet. As described above, the user can shorten or prolong the time necessary for a constant movement (e.g., contact with the touch sensor) in accordance with a driving force of the attachment magnet. Therefore, movement assistance at a higher time accuracy can be achieved by setting the control timing T3 and the end timing T4 in accordance with a driving force.

For example, causing the attachment magnet to apply attraction promotes the contact operation of the user's finger. In this manner, in a case of promoting the movement of the user's finger, the control timing T3 is set to be later as the driving force (attraction) of the attachment magnet increases. For example, adjustment is made to delay the control timing T3 to turn on the electromagnet in a case where the strength of the attraction is higher and to advance the control timing T3 in a case where the strength of the attraction is lower. This enables the timing when the user touches the touch sensor with the finger to be controlled with high accuracy, for example, even in a case where the user's voluntary movement timing is delayed.

Moreover, for example, causing the attachment magnet to apply repulsion suppresses the contact operation of the user's finger. In this manner, in a case of suppress the movement of the user's finger, the end timing T4 is set to be earlier as the driving force (repulsion) of the attachment magnet increases. For example, adjustment is made to advance the end timing T4 to turn off the electromagnet in a case where the strength of the repulsion is higher and to delay the end timing T4 in a case where the strength of the repulsion is lower. This enables the timing when the user touches the touch sensor with the finger to be controlled with high accuracy, for example, in a case where the user's voluntary movement timing is earlier.

Hereinabove, the configuration to drive the body part (finger) of the user through the electromagnet has been mainly described. However, the present technology is not limited thereto. For example, a configuration to drive the body part through a mechanical mechanism may be used. For example, an assistance device including a wearable link mechanism (exoskeleton) moved through a motor or actuator, for example, may be employed. This allows assistance for a movement of each part of the user such as the user's finger, hand, arm, waist, leg, and neck.

In addition, any assistance device capable of moving the body part of the user before the voluntary movement of the user can be used.

Moreover, the present technology can be applied to any movement performed by the user.

As described above, the use of the present technology can perform movement assistance in advance before the body part actually moves, for example, by detecting the spontaneous movement signal. For example, applying the present technology for playing a music instrument or sport, training or assistance for a highly advanced technique, or the like that needs a high temporal resolution can achieve these movements at a high time-accuracy.

For example, the present technology may assist finger movements when playing a music instrument. In this case, for example, an electromagnet or the like provided in an operational portion of the music instrument assists movements of moving the user's fingers closer or further. This allows accurate assistance of a music play timing or the like. Moreover, for example, the use of an assistance system such as an exoskeleton may control a body movement when playing a sport at an appropriate timing.

Moreover, applying the present technology can increase the operation speed in a case where input operations that needs high-speed reactions for an e-sport or the like or quick operations such as touch panel operations of reception is necessary. Moreover, other users may be able to learn skills of users excellent in operation speed.

A signal detecting a change or the like in the surrounding environment of the user may be used as a reference signal for the movement of the body part of the user.

For example, the user needs to perform a driving operation depending on a surrounding environment when driving a vehicle or the like. Such a driving operation may be assisted by detecting changes in surrounding environment.

Movement assistance for accelerating and braking operations by the user's legs is assumed as an example. Such movement assistance is achieved using a mechanism that moves the user's legs by applying attraction or repulsion on magnets attached to, for example, the user's legs (e.g., thighs, shins, ankles), shoe bottoms, or the like, an exoskeleton that moves the user's legs, or the like.

Moreover, the user's vehicle includes, for example, a vehicle-mounted camera that images the front of the vehicle. The vehicle-mounted camera images a video and input it to a computer for image recognition or the like. The computer detects a state of a signal (e.g., a red or blue signal) in front of the vehicle from the video of the vehicle-mounted camera. This detection result is used as a reference signal. Thus, the reference signal can be considered as a signal indicating a sensing result by a predetermined sensor (vehicle-mounted camera) using information for determining whether or not the user will move the user's body part (leg).

A spontaneous movement signal indicating that the user attempts to step on the accelerator has been detected for example in a case where the signal is a red signal as an assumption. In this case, the vehicle may be accelerated irrespective of the red signal. Therefore, movement assistance is executed to avoid the accelerating operation (e.g., removing the user's legs from the gas pedal) before the user steps on the accelerator.

Moreover, a spontaneous movement signal for making a movement of stepping on the brake pedal has not been detected for example in a case where the signal is the red signal as an assumption. In this case, the vehicle may travel ignoring the red signal. Therefore, movement assistance is executed to cause the user to step on the brake pedal so that the vehicle can stop at a suitable position.

The user's erroneous operations and the like can be avoided by using the information to which the user refers when performing an accelerating operation or braking operation as the reference signal as described above.

Moreover, a camera that images the back and sides of the vehicle or the like may be used.

For example, a camera that images the back of the vehicle detects a following vehicle. In this case, when the following vehicle is significantly close, for example, the movement of stepping on the brake pedal is suppressed. This avoids a sudden brake and the like, which can avoid a collision with the following vehicle.

Moreover, for example, A camera that images the side of the vehicle detects a vehicle driving parallel to the vehicle. In this case, an accelerating operation for lane change is suppressed. Alternatively, the user's wheel steering operation and the like may be suppressed. This allows avoidance in advance of a collision and the like with the vehicle driving parallel to the vehicle.

In addition, a distance sensor such as a LiDAR sensor, a radar sensor, and an ultrasonic sensor may be used.

Moreover, for example, a reference signal may be generated on the basis of a video obtained by imaging a periphery of a user playing a sport or the like. For example, in a case where the user plays tennis, a ball trajectory is calculated based on a ball position detected from a video of the periphery of the user. A timing to move the racket is estimated from this result and this information is used as a reference signal. This enables the user to swing the racket at a suitable timing.

Timings for various movements performed by the user can be accurately controlled by detecting necessary information from the surrounding environment of the user and generating the reference signal as described above.

The type of sensor used for generating the reference signal is not limited.

For example, an illuminance sensor that detects brightness of the surrounding environment may be used. This allows movement assistance to suppress the accelerating or braking operation, for example, in a case where the user has a dark field-of-view, for example.

Moreover, weather information or the like based on a road surface sensor, a temperature sensor, or a GPS sensor may be used. This allows detection of a state (e.g., freezing or snowing) of a road surface, for example, for assisting the accelerating or braking operation or the like.

Moreover, a sensor for measuring a wind direction, a wind flow rate, or the like may be used. This enables the user to move considering wind influences or the like, for example, when the user plays a sport such as tennis and golf.

Moreover, an atmospheric sensor, an altitude sensor, or the like may be used. This enables the amount of movement of the user to be controlled by reducing the user's cardiopulmonary burden, for example.

Hereinabove, the case where the computer of the body driving system executes the information processing method according to the present technology has been described. However, another computer capable of communicating with the computer mounted on the body driving system via a network or the like may execute the information processing method and the program according to the present technology.

That is, the information processing method and the program according to the present technology may be executed not only in a computer system configured by a single computer but also in a computer system in which a plurality of computers cooperatively operate. It should be noted that in the present disclosure, the system means a set of a plurality of components (apparatuses, modules (parts), and the like) and it does not matter whether or not all the components are housed in the same casing. Therefore, both a plurality of apparatuses housed in separate casings and connected to one another via a network and a single apparatus having a plurality of modules housed in a single casing are the system.

Executing the information processing method and the program according to the present technology by the computer system includes, for example, both of a case where a single computer executes processing of controlling the action unit that moves the body part of the user and a case where different computers execute these processes. Moreover, executing the respective processes by a predetermined computer includes causing another computer to execute some or all of those processes and acquiring the results.

That is, the information processing method and the program according to the present technology can also be applied to a cloud computing configuration in which a plurality of apparatuses shares and cooperatively processes a single function via a network.

At least two features of the features according to the present technology, which have been described above, may be combined. That is, the various features described in the respective embodiments may be arbitrarily combined across the respective embodiments. Moreover, the above-mentioned various effects are merely exemplary and not limitative, and other effects may be provided.

In the present disclosure, the "same", "equal", "orthogonal", and the like are concepts including "substantially the same", "substantially equal", "substantially orthogonal", and the like. For example, conditions included in a predetermined range (e.g., ±10% range) based on "completely the same", "completely equal", "completely orthogonal", and the like are also included.

It should be noted that the present technology can also take the following configurations.
(1) An information processing apparatus, including
   a control unit that controls an action unit at a third point of time on the basis of a signal indicating a voluntary movement of a user at a first point of time, the action unit being attached to a body part of the user and moving the body part of the user, the third point of time being included in a period from the first point of time to a second point of time, the second point of time being a point of time when a movement of the body part of the user according to the signal indicating the voluntary movement of the user occurs.
(2) The information processing apparatus according to (1), in which the control unit detects a movement signal for making the voluntary movement of the user from the signal indicating the voluntary movement of the user and sets the third point of time to control the action unit, using a detection timing of the movement signal as a basis.
(3) The information processing apparatus according to (2), in which the control unit controls, in a case where the movement signal has been detected, the action unit to accelerate or decelerate the movement of the body part of the user, using as the second point of time as a basis.
(4) The information processing apparatus according to (2) or (3), in which
   the control unit controls the action unit on the basis of a reference signal for the movement of the body part of the user.
(5) The information processing apparatus according to (4), in which
   the reference signal is a signal indicating a timing of the movement of the body part of the user, and
   the control unit sets, on the basis of the timing of the movement of the body part of the user, at least one of the third point of time to control the action unit or a fourth point of time to terminate control on the action unit.
(6) The information processing apparatus according to (5), in which
   the control unit sets at least one of the third point of time or the fourth point of time in accordance with a driving force to drive the action unit.
(7) The information processing apparatus according to (6), in which
   the action unit is capable of driving to promote or suppress the movement of the body part of the user, and
   the control unit sets the third point of time to be later as the driving force of the action unit increases in a case of promoting the movement of the body part of the user and sets the fourth point of time to be earlier as the driving force of the action unit increases in a case of suppressing the movement of the body part of the user.
(8) The information processing apparatus according to any one of (4) to (7), in which
   the reference signal is a signal indicating necessity or unnecessity of a predetermined movement that the user performs by moving the body part of the user, and
   the control unit compares necessity or unnecessity of the predetermined movement indicated by the reference signal with presence or absence of the movement signal for making the predetermined movement and controls the action unit on the basis of the comparison result.
(9) The information processing apparatus according to (8), in which
   the control unit controls the action unit to cause the body part of the user to perform the predetermined movement in a case where the movement signal for making the predetermined movement has not been detected in a situation where the predetermined movement is necessary.
(10) The information processing apparatus according to (8) or (9), in which
   the control unit controls the action unit to prevent the body part of the user from performing the predetermined movement in a case where the movement signal for making the predetermined movement has been detected in a situation where the predetermined movement is unnecessary.
(11) The information processing apparatus according to any one of (4) to (10), in which
   the reference signal is a signal indicating a result of sensing information for the user to determine whether or not to move the body part of the user, the sensing being performed through a predetermined sensor.
(12) The information processing apparatus according to any one of (4) to (10), in which
   the movement of the body part of the user is a movement for performing an input operation for a predetermined application, and
   the reference signal is a signal indicating a timing of the input operation.
(13) The information processing apparatus according to any one of (4) to (10), in which
   the reference signal is a movement signal detected from a signal indicating a voluntary movement of another user different from the user.
(14) The information processing apparatus according to any one of (1) to (13), in which
   the signal indicating the voluntary movement of the user is at least one of an electromyography signal of the user, an electroencephalography signal of the user, or a spinal cord signal of the user.
(15) The information processing apparatus according to any one of (1) to (14), in which
   the action unit is a first magnetic field action unit that receives a force depending on a magnetic field, and
   the control unit controls the first magnetic field action unit by electrically changing a magnetic field between the first magnetic field action unit and a second magnetic field action unit provided separate from the first magnetic field action unit.
(16) The information processing apparatus according to (15), in which
   at least one of the first magnetic field action unit or the second magnetic field action unit includes an electromagnet, and
   the control unit controls the electromagnet to generate a magnetic force between the first magnetic field action unit and the second magnetic field action unit, the magnetic force being attraction or repulsion.
(17) The information processing apparatus according to (15) or (16), in which
   the movement of the body part of the user is a contact movement in which the user touches a movement target with the body part of the user, and
   the second magnetic field action unit is provided in the movement target.
(18) The information processing apparatus according to (17), in which
   the body part of the user is a finger of the user,
   the contact movement is a contact operation with the finger of the user, and
   the movement target is an input apparatus that receives the contact operation.
(19) An information processing method, including
   by a computer system,
   controlling an action unit at a third point of time on the basis of a signal indicating a voluntary movement of a user at a first point of time, the action unit being attached to a body part of the user and moving the body part of the user, the third point of time being included in a period from the first point of time to a second point of time, the second point of time being a point of time when a movement of the body part of the user according to the signal indicating the voluntary movement of the user occurs.
(20) A computer-readable recording medium recording a program to execute
   a step of controlling an action unit at a third point of time on the basis of a signal indicating a voluntary movement of a user at a first point of time, the action unit being attached to a body part of the user and moving the body part of the user, the third point of time being included in a period from the first point of time to a second point of time, the second point of time being a point of time when a movement of the body part of the user according to the signal indicating the voluntary movement of the user occurs.

### Reference Signs List

- S1: spontaneous movement signal
- T1: measurement timing
- T2: movement timing
- T3: control timing
- 1: user
- 2: finger
- 10, 210: attachment magnet unit
- 11: attachment magnet
- 20, 220: electromagnet unit
- 21: electromagnet
- 25: electromagnet driving unit
- 30: body driving unit
- 40, 240: electromyography sensor
- 340: electroencephalography sensor
- 50, 250, 350: electromagnet controller
- 51: storage unit
- 52: electromagnet control unit
- 53: spontaneous movement signal detection unit
- 54: body driving processing control unit
- 100, 200, 300: body driving system

## Claims

1. An information processing apparatus, comprising
a control unit that controls an action unit at a third point of time on a basis of a signal indicating a voluntary movement of a user at a first point of time, the action unit being attached to a body part of the user and moving the body part of the user, the third point of time being included in a period from the first point of time to a second point of time, the second point of time being a point of time when a movement of the body part of the user according to the signal indicating the voluntary movement of the user occurs.

2. The information processing apparatus according to claim 1, wherein
the control unit detects a movement signal for making the voluntary movement of the user from the signal indicating the voluntary movement of the user and sets the third point of time to control the action unit, using a detection timing of the movement signal as a basis.

3. The information processing apparatus according to claim 2, wherein
the control unit controls, in a case where the movement signal has been detected, the action unit to accelerate or decelerate the movement of the body part of the user, using as the second point of time as a basis.

4. The information processing apparatus according to claim 2, wherein
the control unit controls the action unit on a basis of a reference signal for the movement of the body part of the user.

5. The information processing apparatus according to claim 4, wherein
the reference signal is a signal indicating a timing of the movement of the body part of the user, and
the control unit sets, on a basis of the timing of the movement of the body part of the user, at least one of the third point of time to control the action unit or a fourth point of time to terminate control on the action unit.

6. The information processing apparatus according to claim 5, wherein
the control unit sets at least one of the third point of time or the fourth point of time in accordance with a driving force to drive the action unit.

7. The information processing apparatus according to claim 6, wherein
the action unit is capable of driving to promote or suppress the movement of the body part of the user, and
the control unit sets the third point of time to be later as the driving force of the action unit increases in a case of promoting the movement of the body part of the user and sets the fourth point of time to be earlier as the driving force of the action unit increases in a case of suppressing the movement of the body part of the user.

8. The information processing apparatus according to claim 4, wherein
the reference signal is a signal indicating necessity or unnecessity of a predetermined movement that the user performs by moving the body part of the user, and
the control unit compares necessity or unnecessity of the predetermined movement indicated by the reference signal with presence or absence of the movement signal for making the predetermined movement and controls the action unit on a basis of the comparison result.

9. The information processing apparatus according to claim 8, wherein
the control unit controls the action unit to cause the body part of the user to perform the predetermined movement in a case where the movement signal for making the predetermined movement has not been detected in a situation where the predetermined movement is necessary.

10. The information processing apparatus according to claim 8, wherein
the control unit controls the action unit to prevent the body part of the user from performing the predetermined movement in a case where the movement signal for making the predetermined movement has been detected in a situation where the predetermined movement is unnecessary.

11. The information processing apparatus according to claim 4, wherein
the reference signal is a signal indicating a result of sensing information for the user to determine whether or not to move the body part of the user, the sensing being performed through a predetermined sensor.

12. The information processing apparatus according to claim 4, wherein
the movement of the body part of the user is a movement for performing an input operation for a predetermined application, and
the reference signal is a signal indicating a timing of the input operation.

13. The information processing apparatus according to claim 4, wherein
the reference signal is a movement signal detected from a signal indicating a voluntary movement of another user different from the user.

14. The information processing apparatus according to claim 1, wherein
the signal indicating the voluntary movement of the user is at least one of an electromyography signal of the user, an electroencephalography signal of the user, or a spinal cord signal of the user.

15. The information processing apparatus according to claim 1, wherein
the action unit is a first magnetic field action unit that receives a force depending on a magnetic field, and
the control unit controls the first magnetic field action unit by electrically changing a magnetic field between the first magnetic field action unit and a second magnetic field action unit provided separate from the first magnetic field action unit.

16. The information processing apparatus according to claim 15, wherein
at least one of the first magnetic field action unit or the second magnetic field action unit includes an electromagnet, and
the control unit controls the electromagnet to generate a magnetic force between the first magnetic field action unit and the second magnetic field action unit, the magnetic force being attraction or repulsion.

17. The information processing apparatus according to claim 15, wherein
the movement of the body part of the user is a contact movement in which the user touches a movement target with the body part of the user, and
the second magnetic field action unit is provided in the movement target.

18. The information processing apparatus according to claim 17, wherein
the body part of the user is a finger of the user,
the contact movement is a contact operation with the finger of the user, and
the movement target is an input apparatus that receives the contact operation.

19. An information processing method, comprising
by a computer system,
controlling an action unit at a third point of time on a basis of a signal indicating a voluntary movement of a user at a first point of time, the action unit being attached to a body part of the user and moving the body part of the user, the third point of time being included in a period from the first point of time to a second point of time, the second point of time being a point of time when a movement of the body part of the user according to the signal indicating the voluntary movement of the user occurs.

20. A computer-readable recording medium recording a program to execute
a step of controlling an action unit at a third point of time on a basis of a signal indicating a voluntary movement of a user at a first point of time, the action unit being attached to a body part of the user and moving the body part of the user, the third point of time being included in a period from the first point of time to a second point of time, the second point of time being a point of time when a movement of the body part of the user according to the signal indicating the voluntary movement of the user occurs.
